# EUROPÄISCHE PATENTANMELDUNG

(11) **EP 1 621 631 A1**
(43) Veröffentlichungstag der Anmeldung: **01.02.2006**
(21) Anmeldenummer: 05016436.7
(22) Anmeldetag: 28.07.2005
(51) Int. Cl.: C12N 15/82, C12N 9/02, C12N 15/53

(54) **Verfahren zur Herstellung von C6- und/oder C9-Aldehyden und -Alkoholen in Pflanzen durch 9/13-Divinylethersynthase**

(30) Priorität: 28.07.2004 DE 102004036466
(71) Anmelder: Georg-August-Universität Göttingen, 37073 Göttingen (DE)
(72) Erfinder: Feussner, Ivo, 37085 Göttingen (DE); Stumpe, Michael, 37083 Göttingen (DE); Carsjens, Jan-Gerrit, 37081 Göttingen (DE)
(74) Vertreter: Neuefeind, Regina

(57) **Zusammenfassung**

Die vorliegende Erfindung betrifft ein Verfahren zur Herstellung von C₆- und/oder C₉₋Aldehyden, -Alkoholen sowie deren Ester, das auf der Umsetzung von Fettsäurehydroperoxiden mit rekombinanter 9/13-Divinylethersynthase (9/13-DES) basiert. Des Weiteren betrifft die Erfindung ein Verfahren zur Herstellung von Pflanzen mit erhöhter Pathogenresistenz durch die transgene Expression der 9/13-Divinylethersynthase sowie die Applikation von Divinylethern, die *in vitro* mit Hilfe einer 9/13-Divinylethersynthase hergestellt wurden, an Pflanzen, um eine Pathogenresistenz zu vermitteln.

## Beschreibung

Die vorliegende Erfindung betrifft ein Verfahren zur Herstellung von C₆- und/oder C₉₋Aldehyden, -Alkoholen sowie deren Ester, das auf der Umsetzung von Fettsäurehydroperoxiden mit rekombinanter 9/13-Divinylethersynthase (9/13-DES) basiert. Des Weiteren betrifft die Erfindung ein Verfahren zur Herstellung von Pflanzen mit erhöhter Pathogenresistenz durch die transgene Expression der 13-Divinylethersynthase sowie die Applikation von Divinylethern, die *in vitro* mit Hilfe einer 13-Divinylethersynthase hergestellt wurden, an Pflanzen, um eine Pathogenresistenz zu vermitteln.

Mehrfach ungesättigte Fettsäuren wie z.B. Linol- oder Linolensäure können durch die Wirkung der Enzyme der Lipoxygenase-Familie, ubiquitär in Pflanzen, Tieren und Mikroorganismen vorkommenden Enzymen, die die Oxygenierung des (1Z, 4Z)-Pentadiensystems von mehrfach ungesättigten Fettsäuren katalysieren, zu Oxylipinen oxygeniert werden. Die Hydroperoxidgruppe kann durch die Enzyme 9-LOX bzw. 13-LOX an zwei verschiedenen Positionen in die ungesättigten Fettsäuren eingeführt werden. Dadurch entstehen im Falle der Linolsäure (9S, 10E, 12Z)-9-Hydroperoxy-10,12-Octadecadiensäure (9-HPOD) oder (13S, 9Z, 11E)-13-Hydroperoxy-9,11-Octadecadiensäure (13-HPOD) und im Falle der Linolensäure (9S, 10E, 12Z, 15Z)-9-Hydroperoxy-10,12,15-Octadecatriensäure (9-HPOT) sowie (9Z, 11E, 13S, 15Z)-13-Hydroperoxy-9,11,15-Octadecatriensäure (13-HPOT).

Die Produkte der Lipoxygenasereaktion dienen in Pflanzen als Substrate für eine Vielzahl von Enzymen, von denen die wichtigsten die Allenoxidsynthase, die Hydroperoxid-Lyase und die Divinylethersynthase sind. Diese Enzyme gehören alle zur Klasse der Cytochrom-P450-haltigen Enzyme mit einem Häm-Molekül als prosthetischer Gruppe. Durch die Aktivität dieser Enzyme werden viele wichtige Hormone wie beispielsweise Jasmonsäure und Traumatin sowie Geschmacks- und Duftmoleküle in Pflanzen, beispielsweise Hexenale, (3Z)-Hexenol und Nonenale, gebildet.

Der Allenoxidsynthase (AOS)-Reaktionsweg führt zur Biosynthese von Jasmonsäure, die an der Regulation von Stress- und Krankheitsresistenzantworten beteiligt ist, indem sie die Transkription bestimmter Gene induziert und die Translation von Jasmonat-induzierten Proteinen reguliert.

Fettsäurehydroperoxid-Lyase (HPL) katalysiert die Spaltung von Kohlenstoff-Kohlenstoff-Bindungen in mehrfach ungesättigten Fettsäurehydroperoxiden, wodurch z.B. aus C₁₈₋Fettsäurehydroperoxiden kurzkettige C₆- bzw. C₉-Aldehyde und w-Oxosäuren der Kettenlänge C₁₂ bzw. C₉ entstehen (Vick et al. (1976) Plant Physiol. 57: 780-788). Die kurzkettigen flüchtigen C₆-Aldehyde liefern einen Beitrag zu den sogenannten "grünen Duftnoten" in einer Vielzahl von Pflanzenblättern, Gemüsen und Früchten. Diese Duftnoten werden häufig auch als "frisches Gras" bezeichnet. Andere kurzkettige flüchtige Aldehyde, wie beispielsweise (3Z,6Z)-Nonadienal, der gemäß dem Stand der Technik durch Spaltung von (9S, 10E, 12Z, 15Z)-9-Hydroperoxy-10, 12, 15-Octadecatriensäure durch eine 9-Hydroperoxid-Lyase (9-HPL) erzeugt wird, liefern ein Melonen- oder Gurken-artiges Aroma und/oder einen entsprechenden Geschmacksbeitrag in Früchten und Gemüse. Derartige Merkmale sind für Duft- und Aromastoffe, insbesondere für die Lebensmittelindustrie, aber auch für die Kosmetik, pharmazeutische und chemische Industrie, von großer Bedeutung.

Ferner wird angenommen, dass kurzkettige Aldehyde ebenfalls eine Rolle bei der Pathogenresistenz spielen. Beispielsweise haben Croft et al. (1993) Plant Physiol. 101: 13-24, berichtet, dass der Gehalt an (3Z)-Hexenol und (2E)-Hexenal während einer hypersensitiven Antwort (HR) der Ackerbohne anstieg. Ferner wurde gezeigt, dass (2E)-Hexenal ein wirksames antibakterielles Mittel ist. Außerdem wurde beschrieben, dass durch die Behandlung von Pflanzen mit (2E)-Hexenol verschiedene Gene aktiviert werden, die zur Pathogenabwehr beitragen (Gomi et al. (2003) Plant Mol Biol. 53 (1-2): 189-199; Gomi et al. (2003) J Plant Physiol. 160 (10): 1219-1231).

Ein drittes wichtiges Enzym für die Umsetzung der Fettsäurehydroperoxide ist die Divinylethersynthase (DES), die die Fettsäurehydroperoxide zu konjugierten Etherfettsäuren umsetzt, die ein Sauerstoffatom innerhalb der Kohlenwasserstoffkette enthalten. In der Kartoffel führt beispielsweise die sequentielle Wirkung einer 9-Lipoxygenase und einer Divinylethersynthase, die für 9-Hydroperoxide spezifisch ist (9-DES), zur Bildung der Divinylether Colnelsäure bzw. Colnelensäure aus Linolsäure bzw. Linolensäure.

Die enzymatische Aktivität der Divinylethersynthasen wurde das erste Mal in Extrakten aus Kartoffelsuspensionskulturen nachgewiesen. Es konnte gezeigt werden, dass bei Inkubation von 9-HPOD/9-HPOT mit Kartoffelextrakten die Divinylether Colnelsäure und Colnelensäure gebildet werden (Galliard und Phillips (1972) Biochem J. 129: 743 - 753). Mittlerweile wurden Divinylethersynthasen, die spezifisch 9S-Hydroperoxide, aber nicht oder nur in geringem Umfang 13S-Hydroperoxide umsetzen, aus der Tomate und der Kartoffel kloniert (Itoh und Howe (2001) J. Biol. Chem. 276: 3620 - 3627; WO 02/29018 A2; Stumpe et al. (2001) FEBS Lett. 507: 371 - 376; DE 101 29 338 A1). Ebenfalls kloniert wurde bereits eine Divinylethersynthase aus dem Moos *Physcomitrella patens,* die spezifisch sowohl für 9-HPOD/9-HPOT als auch für 13-HPOD/13-HPOT ist (WO 03/006647).

Auch in Knoblauch *(Allium sativum)* konnte die Bildung der Divinylether Etherolsäure bzw. Etherolensäure aus 13-HPOD bzw. 13-HPOT beobachtet werden (Grechkin et al. (1995) FEBS Lett. 371: 159-162). Bisher ist es aber noch nicht gelungen, eine 13-DES aus höheren Pflanzen zu isolieren, weshalb die Produkte der 13-DES, Etherol- und Etherolensäure, nur durch Inkubation der Substrate 13-HPOD bzw. 13-HPOT mit Knoblauchknollenextrakt erhalten werden können. Dabei kommt jedoch eine Mischung aus verschiedensten Enzymen in einer nicht überschaubaren Wechselwirkung zum Einsatz.

Für die Divinylether Colnelsäure und Colnelensäure, die durch die Aktivität einer 9-DES gebildet werden, konnte bereits gezeigt werden, dass Sie nach Infektion von Kartoffelblättern mit *Phytophtora infestans* in größeren Mengen akkumulieren und daher möglicherweise zur Vermittlung der Pathogenresistenz beitragen (Weber et al. (1999) Plant Cell 11: 485 - 493; Göbel et al. (2001) J. Biol. Chem. 276: 6267-6273; Göbel et al. (2002) Biochim Biophys Acta 1584 (1): 55 - 64).

Die Charakterisierung von an der Oxylipin-Biosynthese beteiligten Enzymen ist für die weitere Untersuchung des Pflanzenfettsäuremetabolismus und für die Entwicklung von transgenen Pflanzen mit veränderten Aroma- und Geschmackseigenschaften von großer Bedeutung. Dadurch können die Aroma- und Geschmackseigenschaften von Pflanzen, die für die Lebensmittelindustrie von Bedeutung sind, verstärkt, gesteuert, modifiziert oder auf andere Art und Weise verändert werden. Ferner ist die Aufklärung der physiologischen Rollen von Enzymen der Oxylipin-Biosynthese und ihrer Produkte von Interesse für die weitere Untersuchung von Krankheitsresistenzen.

Die Biosynthese von C₆- und C₉-Aldehyden und den dazu gehörenden C₁₂- und C₉-ω-Keto-Fettsäuren aus Linol- oder Linolensäure ist eine weitverbreitete Reaktion im Pflanzenreich (z.B. K. Matsui, Belgian J. Bot. (1998), 131: 50-62). Beide Substanzgruppen können in der Pflanze weiteren Umsetzungen unterliegen. So werden die Aldehyde durch Dehydrogenasen zu den entsprechenden Alkoholen und die Fettsäuren zu ω-Hydroxy-Fettsäuren reduziert.

Die so erzeugten C₆- bzw. C₉-Alkohole können ferner mit Säuren, insbesondere Essigsäure, zu den entsprechenden Estern, insbesondere Essigsäurestern umgesetzt werden, die ebenfalls von großer industrieller Bedeutung als Duft- und Aromastoffe sind.

Die Synthese der C₉-Aldehyde (3Z)-Nonenal bzw. (3Z,6Z)-Nonadienal beginnt mit der Umsetzung von Linol- bzw. α-Linolensäure mit 9-Lipoxygenase zu (9S)-Hydroperoxy-Derivaten. Diese können anschließend durch eine 9-Hydroperoxid-Lyase (9-HPL) in die C₉₋ω-Keto-Fettsäure und (3Z)-Nonenal bzw. (3Z,6Z)-Nonadienal gespalten werden. Die entstandenen Aldehyde können dann enzymatisch bzw. chemisch zu den (2E)-Derivaten umgesetzt werden und/oder zu den C₉-Alkoholen (3Z)-Nonenol bzw. (3Z,6Z)-Nonadienol reduziert werden.

Die Synthese der C₆-Aldehyde (3Z)-Hexenal bzw. -Hexanal beginnt mit der Umsetzung von Linol- bzw. α-Linolensäure mit 13-Lipoxygenase zu (13S)-Hydroperoxy-Derivaten. Diese können anschließend durch eine 13-Hydroperoxid-Lyase (13-HPL) in die C₁₂-ω-Oxo-Fettsäure und (3Z)-Hexenal bzw. -Hexanal gespalten werden. Die entstandenen Aldehyde können dann enzymatisch bzw. chemisch zu den (2E)-Derivaten umgesetzt werden und/oder zu den C₆-Alkoholen (3Z)-Hexenol bzw. -Hexanol reduziert werden.

N.J. Bate et al., Plant Physiology (1998), 117: 1393-1400, beschreiben die molekulare Charakterisierung eines für eine 13-Hydroperoxid-Lyase kodierenden Gens aus *Arabidopsis thaliana.* Dabei wurde eine deutliche HPL-Aktivität beobachtet, wenn (13S,9Z,11E,15Z)-13-Hydroperoxy-9,11,15-Octadecatriensäure als Substrat verwendet wurde, während die Aktivität mit (13S,9Z,1 1E)-13-Hydroperoxy-9,11-Octadecadiensäure etwa um eine Größenordnung niedriger war.

Die internationale Patentanmeldung WO 00/22145 beschreibt ein Hydroperoxid-Lyase-Gen aus Mais. Ferner sind dort Verfahren zur Verbesserung von Krankheitsresistenzen und zur Änderung des Gehaltes von Aromamolekülen in Pflanzen offenbart. Diese Verfahren umfassen die Expression von Hydroperoxid-Lyase-Genen in Pflanzen, Pflanzenzellen und Pflanzengeweben.

Die im Stand der Technik beschriebenen Verfahren beschreiben somit die Spaltung von 13-Hydroperoxidfettsäuren durch 13-Hydroperoxid-Lyase bzw. 9-Hydroperoxidfettsäuren durch 9-Hydroperoxid-Lyase. D.h., die Herstellung eines C₆- bzw. C₉-Aldehyds bzw. -Alkohols aus einer C₁₈-Fettsäure erfordert gemäß dem Stand der Technik die sequentielle Umsetzung mit einer Lipoxygenase zur Erzeugung des entsprechenden Hydroperoxid-Fettsäurederivats sowie mit einer Hydroperoxid-Lyase zur Spaltung des Hydroperoxid-Fettsäurederivats.

Zur Herstellung von Duft- und Aromastoffen ist es von wesentlicher Bedeutung, die jeweiligen Bestandteile in möglichst hoher Reinheit einzusetzen. Die aus der durch die Hydroperoxid-Lyase katalysierte Umsetzung von Hydroperoxid-Derivaten der α-Linolensäure erzeugten Aldehyde weisen jedoch den Nachteil auf, dass die (3Z)-Doppelbindung säurelabil ist und somit Mischungen aus (3Z)-, (2E)- und (3E)-Isomeren entstehen können. Derartige Mischungen sind für die Weiterverarbeitung zu entsprechenden Alkoholen und Estern und insbesondere für die Verwendung der Aldehyde, Alkohole und Ester als Bestandteile von Duft- und Aromastoffen unerwünscht.

DE 101 29 338 A1 beschreibt ein Verfahren zur Herstellung von C₉-Aldehyden und -Alkoholen sowie Estern der Alkohole durch Expression einer 9-DES und anschließendes Ansäuern der entstandenen Divinylether, wodurch diese zu den Aldehyden zerfallen. Mit diesem Verfahren ist es aber nicht möglich, C₆-Aldehyde, -Alkohole und Ester der Alkohole herzustellen.

Aufgabe der vorliegenden Erfindung ist es daher, ein Verfahren zur Herstellung von C₆- und/oder C₉-Aldehyden, -Alkoholen sowie Estern der Alkohole bereitzustellen, bei dem die Aktivität einer 9- oder 13-Hydroperoxid-Lyase nicht benötigt wird. Insbesondere ist es eine Aufgabe der vorliegenden Erfindung, ein Verfahren zur Herstellung von stabilen (2E)-Isomeren von C₆- und/oder C₉-Aldehyden, -Alkoholen sowie Estern der Alkohole bereitzustellen.

Ferner ist es eine Aufgabe der vorliegenden Erfindung, transgene Pflanzen bzw. Pflanzenzellen zur Verfügung zu stellen, die eine erhöhte Resistenz gegenüber Pathogenen aufweisen.

Weiterhin ist es eine Aufgabe der vorliegenden Erfindung, Verfahren zur Verfügung zu stellen, mit denen Divinylether *in vitro* synthetisiert werden können und zur Applikation an Pflanzen verwendet werden können.

Diese Aufgaben werden durch die Gegenstände der unabhängigen Ansprüche gelöst. Vorteilhafte Ausgestaltungen sind in den Unteransprüchen definiert.

Es ist nun erstmals gelungen, eine 9/13-DES aus Knoblauch zu klonieren, die sowohl 13-Hydroperoxide als auch 9-Hydroperoxide umsetzt, weshalb neben den 13-Divinylethern sowie C₆-Aldehyden,- Alkoholen und Estern der Alkohole auch 9-Divinylether sowie C₉₋Aldehyde, -Alkohole sowie deren Ester durch die Aktivität dieses Enzyms entstehen können Die Aldehyde entstehen aus den Ethern dadurch, dass die Ether durch mildes Ansäuern in die entsprechenden Aldehyde zerfallen. Schließlich sind die Divinyletherprodukte Etherol-, Etherolen-, Colnel- und Colnelensäure dieses Enzyms auch an der Pathogenabwehr in Pflanzen beteiligt.

Die genannten Aufgaben der vorliegenden Erfindung werden daher im Wesentlichen dadurch gelöst, dass ein isoliertes Nukleinsäuremolekül zur Verfügung gestellt wird, das für eine 9/13-Divinylethersynthase kodiert und daher die Herstellung von 13- und/oder 9-Divinylethern sowie C₆- und/oder C₉-Aldehyden, -Alkoholen und Estern dieser Alkohole ermöglicht. Ferner vermitteln die Divinylether, die durch die 9/13-DES gebildet werden, eine erhöhte Pathogenresistenz in Pflanzen.

Gegenstand der vorliegenden Erfindung ist daher ein isoliertes Nukleinsäuremolekül, enthaltend eine Nukleinsäuresequenz ausgewählt aus der Gruppe bestehend aus:
i) DNA-Sequenzen umfassend Nukleotidsequenzen, die von SEQ ID No. 1 oder Fragmenten dieser Sequenz kodiert werden,
ii) DNA-Sequenzen umfassend Nukleotidsequenzen, die für ein Protein mit der in SEQ ID No. 2 angegebenen Aminosäuresequenz oder Fragmente davon kodieren,
iii) DNA-Sequenzen umfassend Nukleotidsequenzen, die zu der in SEQ ID No. 1 angegebenen Nukleotidsequenz eine Sequenzidentität von mindestens 60% aufweisen, und/oder
iv) DNA-Sequenzen umfassend Nukleotidsequenzen, die unter stringenten Bedingungen mit einem komplementären Strang einer Nukleotidsequenz von i) bis iii) hybridisieren,

die für ein Protein mit der Aktivität einer 13-Divinylethersynthase kodiert.

Bevorzugt stammt die Nukleinsäuresequenz aus *Allium sativum.*

Die im Verfahren verwendeten Nukleinsäuremoleküle, z.B. ein Nukleinsäuremolekül mit einer Nukleotidsequenz der SEQ ID No. 1, die für ein Protein mit der Aktivität einer 9/13-DES kodiert, oder einem Teil dieser Sequenz, können unter Verwendung molekularbiologischer Standardtechniken und der hier bereit gestellten Sequenzinformation isoliert werden. Auch kann mit Hilfe von Vergleichsalgorithmen, wie sie z.B. auf der NCBI-Homepage unter http://www.ncbi.nlm.nih.gov zu finden sind, beispielsweise eine homologe Sequenz oder homologe, konservierte Sequenzbereiche auf DNA- oder Aminosäureebene identifiziert werden. Wesentliche Teile dieser Sequenz oder die gesamte homologe Sequenz können als Hybridisierungssonde unter Verwendung von Standardhybridisierungstechniken (wie z.B. beschrieben in Sambrook et al., vide supra) zur Isolierung weiterer im Verfahren nützlicher Nukleinsäuresequenzen aus anderen Organismen durch das Screening von cDNA- und/oder genomischen Banken verwendet werden. Überdies lässt sich ein Nukleinsäuremolekül, umfassend eine vollständige Sequenz der SEQ ID No. 1 oder einen Teil davon, durch Polymerasekettenreaktion isolieren, wobei Oligonukleotidprimer auf der Basis der hier angegebenen Sequenzen oder von Teilen davon verwendet werden (z.B. kann ein Nukleinsäuremolekül, umfassend die vollständige Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18: 5294 - 5299) und daraus mittels reverser Transkriptase (z.B. Moloney-MLV-Reverse Transkriptase, erhältlich von Gibco/BRL, Bethesda, MD oder AMV-Reverse-Transkriptase, erhältlich von Seikagaku Amerika, Inc., St. Petersburg, FL) cDNA herstellen. Synthetische Oligonukleotidprimer zur Amplifizierung mittels Polymerasekettenreaktion lassen sich auf der Basis der in SEQ ID No. 1 dargestellten Sequenz oder mit Hilfe der in SEQ ID No. 2 dargestellten Aminosäuresequenz erstellen. Eine erfindungsgemäße Nukleinsäure kann unter Verwendung von cDNA oder alternativ von genomischer DNA als Matrize und geeigneten Oligonukleotidprimern mittels Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer 9/13-DES-Nukleotidsequenz entsprechen, können durch Standardsyntheseverfahren, beispielsweise mit einem automatischen DNA-Synthesegerät, hergestellt werden. Die 9/13-DES-Aktivität der durch diese Nukleinsäuresequenzen kodierten Proteine lässt sich dann durch Enzymtests, wie sie in den Beispielen der vorliegenden Anmeldung beschrieben sind, bestimmen.

Unter dem Begriff "Fragmente der DNA", wie er hier verwendet wird, versteht man Teilstücke der DNA, die für ein Protein kodieren, das die Aktivität einer 9/13-DES besitzt, wobei die von den DNA-Teilstücken kodierten Proteine im wesentlichen die gleiche 9/13-DES-Aktivität aufweisen wie die von der vollständigen DNA-Sequenz kodierten Proteine und sich mit diesen Fragmenten die erfindungsgemäße Erhöhung der Pathogenresistenz in transgenen Pflanzen und/oder die Erzeugung von C₆- und/oder C₉-Aldehyden, -Alkoholen sowie den Estern der Alkohole erzielen lässt.

Der Begriff "Hybridisierung unter stringenten Bedingungen" bedeutet im Zusammenhang dieser Erfindung, dass die Hybridisierung *in vitro* unter Bedingungen durchgeführt wird, die stringent genug sind, um eine spezifische Hybridisierung zu gewährleisten. Stringente in vitro-Hybridisierungsbedingungen sind dem Fachmann bekannt und können der Literatur entnommen werden (z.B. Sambrook und Russell (2001) Molecular Cloning: A Laboratory Manual, 3. Auflage, Cold Spring Harbour Laboratory Press, Cold Spring Harbour, NY). Der Begriff "spezifische Hybridisierung" bezieht sich auf den Umstand, dass ein Molekül unter stringenten Bedingungen präferentiell an eine bestimmte Nukleinsäuresequenz, die Zielsequenz, bindet, wenn diese Teil einer komplexen Mischung von z.B. DNA- oder RNA-Molekülen ist, aber nicht oder zumindest wesentlich reduziert an andere Sequenzen.

Stringente Bedingungen sind von den Umständen abhängig. Längere Sequenzen hybridisieren spezifisch bei höheren Temperaturen. Generell werden stringente Bedingungen so ausgewählt, dass die Hybridisierungstemperatur circa 5°C unter dem Schmelzpunkt (Tₘ) für die spezifische Sequenz bei einer definierten Ionenstärke und einem definierten pH liegt. Die Tₘ ist die Temperatur (bei einem definierten pH-Wert, einer definierten Ionenstärke und einer definierten Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionenkonzentration (oder Konzentration eines anderen Salzes) bei einem pH zwischen 7,0 und 8,3 und die Temperatur mindestens 30°C für kurze Moleküle (d.h. z.B. 10 bis 50 Nukleotide) beträgt. Zusätzlich können stringente Bedingungen die Zugabe von Stoffen wie z.B. Formamid, das die Hybride destabilisiert, umfassen. Bei einer Hybridisierung unter stringenten Bedingungen, wie hier verwendet, bleiben gewöhnlich Nukleotidsequenzen, die mindestens 60% homolog zueinander sind, aneinander hybridisiert. Vorzugsweise sind die stringenten Bedingungen derart gewählt, dass Sequenzen, die mindestens etwa 65%, bevorzugt mindestens etwa 70% und besonders bevorzugt mindestens etwa 75% oder stärker zueinander homolog sind, gewöhnlich aneinander hybridisiert bleiben. Ein bevorzugtes, nichteinschränkendes Beispiel für stringente Hybridisierungsbedingungen sind Hybridisierungen in 6 x Natriumchlorid/Natriumcitrat (SSC) bei etwa 45°C, gefolgt von einem oder mehreren Waschschritten in 0,2 x SSC, 0,1% SDS bei 50 bis 65°C. Die Temperatur schwankt beispielsweise unter Standardhybridisierungsbedingungen je nach dem Typ der Nukleinsäure zwischen 42°C und 58°C in wässrigem Puffer mit einer Konzentration von 0,1 bis 5 x SSC (pH 7,2).

Falls im oben genannten Puffer organisches Lösungsmittel, z.B. 50% Formamid, vorliegt, liegt die Temperatur unter Standardbedingungen bei etwa 42°C. Vorzugsweise sind die Hybridisierungsbedingungen für DNA:DNA-Hybride zum Beispiel 0,1 x SSC und 20°C bis 45°C, vorzugsweise 30°C bis 45°C. Vorzugsweise sind die Hybridisierungsbedingungen für DNA:RNA-Hybride zum Beispiel 0,1 x SSC und 30°C bis 55°C, vorzugsweise zwischen 45°C bis 55°C. Die vorstehend genannten Hybridisierungstemperaturen sind beispielsweise für eine Nukleinsäure mit etwa 100 Basenpaaren Länge und einem G/C-Gehalt von 50% in Abwesenheit von Formamid bestimmt. Der Fachmann weiß, wie die erforderlichen Hybridisierungsbedingungen anhand von Lehrbüchern, wie den vorstehend erwähnten oder den folgenden Lehrbüchern Current Protocols in Molecular Biology, John Wiley & Sons, N.Y. (1989), Hames und Higgins (Hrsgb.) 1985, Nucleic Acids Hybridization: A Practical Approach, IRL Press at Oxford University Press, Oxford; Brown (Hrsgb.) 1991, Essential Molecular Biology: A Practical Approach, IRL Press at Oxford University Press, Oxford, bestimmt werden können.

Der Begriff "Sequenzidentität" im Sinne der Erfindung bezeichnet eine Identität, also gleiche Nukleotide in gleicher 5'-3'-Reihenfolge, über die gesamte Sequenz zu der in SEQ ID No. 1 angegebenen Nukleinsäuresequenz von mindestens 60%, bevorzugt von mindestens 70, 75 und 80%, besonders bevorzugt von mindestens 85 und 90% und am meisten bevorzugt von mindestens 92, 94, 96 und 98%.

Die Sequenzidentität wird über eine Reihe von Programmen, die auf verschiedenen Algorithmen beruhen, bestimmt. Dabei liefern die Algorithmen von Needleman und Wunsch oder Smith und Waterman besonders zuverlässige Ergebnisse. Für die Sequenzvergleiche wurde das Programm PileUp (Feng und Doolittle (1987) J. Mol. Evolution 25: 351- 360; Higgins et al. (1989) CABIOS 5: 151 - 153) oder die Programme Gap und Best Fit (Needleman und Wunsch (1970) J. Mol. Biol. 48: 443 - 453 und Smith und Waterman (1981) Adv. Appl. Math. 2: 482 - 489) verwendet, die im GCG-Software-Paket (Genetics Computer Group, 575 Science Drive, Madison, Wisconsin, USA) enthalten sind.

Die oben in Prozent angegebenen Sequenzidentitätswerte wurden mit dem Programm GAP über den gesamten Sequenzbereich mit folgenden Einstellungen ermittelt: Gap Weight: 50, Length Weight: 3, Average Match: 10000 und Average Mismatch: 0,000.

Diese Einstellungen wurden, falls nicht anders angegeben, als Standardeinstellungen für Sequenzvergleiche verwendet.

Nukleinsäuresequenzen, die von der in SEQ ID No.1 angegebenen Nukleinsäuresequenz abweichen, können z.B. durch Einbringen einer oder mehrerer Nukleotidsubstitutionen, -additionen oder -deletionen in eine Nukleotidsequenz der SEQ ID No. 1 erzeugt werden, so dass Proteine entstehen, in die gegenüber der in SEQ ID No. 2 angegebenen Sequenz eine oder mehrere Aminosäuresubstitutionen, -additionen oder -deletionen eingebracht wurden. Mutationen können in eine der Sequenzen der SEQ ID No. 1 durch Standardtechniken, wie z.B. stellenspezifische Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste, also an Aminosäureresten, die die enzymatische Aktivität der 9/13-DES nicht beeinflussen, hergestellt. Bei einer "konservativen Aminosäuresubstitution" wird ein Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure und Glutaminsäure), ungeladenen polaren Seitenketten (z.B. Glycin, Asparagin, Glutamin, Serin, Threonin, Tyrosin, Cystein), unpolaren Seitenketten (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan). Ein vorhergesagter nicht-essentieller Aminosäurerest in der erfindungsgemäßen 9/13-DES wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der für die 9/13-DES kodierenden Sequenz eingebracht werden, z.B. durch Sättigungsmutagenese, und die resultierenden Mutanten können nach der 9/13-DES-Aktivität durchmustert werden, indem das kodierte Protein rekombinant exprimiert wird, um Mutanten zu identifizieren, die die 9/13-DES-Aktivität beibehalten haben. Die 9/13-DES-Aktivität des Proteins kann z.B. unter Verwendung der in den Beispielen beschriebenen Tests bestimmt werden. Es ist aber auch möglich, Mutanten herzustellen, die nicht mehr den normalerweise in der Zelle herrschenden Regulationsmechanismen wie der allosterischen Regulation oder der kovalenten Modifizierung unterliegen. Dadurch können konstitutiv aktive Enzyme hergestellt werden. Des weiteren können Mutanten hergestellt werden, die eine veränderte Substrat- oder Produktspezifität aufweisen (Hornung et al. (1999), Proc. Natl. Acad. Sci. USA 96:4192-4197). Weiterhin können Mutanten hergestellt werden, die ein verändertes Aktivitäts-, Temperatur- und/oder pH-Profil aufweisen.

Ein weiterer Gegenstand der vorliegenden Erfindung sind Proteine oder Proteinfragmente, die durch die vorhergehend genannten Nukleinsäuremoleküle kodiert werden.

Der Begriff "Fragmente des Proteins", wie er hier verwendet wird, bezeichnet Teilstücke des Proteins, das die Aktivität einer 9/13-DES besitzt, wobei die Teilstücke des Proteins im wesentlichen die gleiche 9/13-DES-Aktivität aufweisen wie das Protein in seiner vollen Länge und sich mit diesen Fragmenten die erfindungsgemäße Herstellung von 9- und/oder 13-Divinylethern erzielen lässt.

Unter "im Wesentlichen gleicher enzymatischer Aktivität" der im erfindungsgemäßen Verfahren verwendeten 9/13-DES ist zu verstehen, dass die enzymatische Aktivität gegenüber den durch die Sequenz mit SEQ ID No. 1 und deren Derivate kodierten Enzymen im Vergleich noch mindestens 50%, bevorzugt mindestens 60%, besonders bevorzugt mindestens 70% und ganz besonders bevorzugt mindestens 80% und am meisten bevorzugt mindestens 90% beträgt. 9/13-DES-Enzyme mit im Wesentlichen gleicher enzymatischer Aktivität sind damit ebenfalls geeignet, in Wirtszellen die Herstellung von 9- und/oder 13-Divinylethern zu bewirken.

Ebenfalls Gegenstand der Erfindung sind rekombinante Nukleinsäuremoleküle, die eine der vorstehend genannten Nukleinsäuresequenzen sowie geeignete Regulations- und/oder Signalsequenzen umfassen. Dabei können diese Regulations- und/oder Signalsequenzen sowohl die Expression in prokaryontischen als auch in eukaryontischen Zellen steuern. Geeignete Regulations- und Signalsequenzen sind dem Fachmann wohl bekannt und können zudem der Literatur entnommen werden.

Gegenstand der vorliegenden Erfindung sind ebenfalls Mikroorganismen und transgene Pflanzenzellen bzw. Pflanzen, die ein rekombinantes Nukleinsäuremolekül enthalten und im Vergleich zum Wildtyp einen erhöhten Gehalt einer 9/13-Divinylethersynthase aufweisen. Vorzugsweise beträgt diese Erhöhung des Gehalts mindestens 5%, bevorzugt mindestens 20%, ebenfalls bevorzugt mindestens 50%, besonders bevorzugt mindestens 100%, ebenfalls besonders bevorzugt mindestens den Faktor 5, insbesondere bevorzugt mindestens den Faktor 10, ebenfalls insbesondere bevorzugt mindestens den Faktor 50 und am meisten bevorzugt den Faktor 100.

Unter einem "Wildtyp" wird erfindungsgemäß der entsprechende nicht genetisch veränderte Ausgangsorganismus verstanden.

Erfindungsgemäß wird außerdem ein Verfahren zur Herstellung von C₆- und/oder C₉₋Aldehyden bzw. C₆- und/oder C₉-Alkoholen bzw. Estern der C₆- und/oder C₉-Alkohole aus mehrfach ungesättigten Fettsäuren der Kettenlänge C₁₈, die zumindest Doppelbindungen an den Positionen Δ9 und Δ12 aufweisen, bereit gestellt, umfassend die folgenden Schritte:
a) die Bereitstellung eines rekombinanten Nukleinsäuremoleküls, das für ein Protein mit der Aktivität einer 9/13-DES kodiert;
b) die Übertragung des Nukleinsäuremoleküls aus a) auf eukaryontische oder prokaryontische Wirtszellen,
c) die Expression der rekombinanten 9/13-Divinylethersynthase in den Zellen;
d) die Umsetzung von 9- und/oder 13-Hydroperoxiden der Fettsäuren mit 9/13-Divinylethersynthase zu Divinylethern;
e) die Umsetzung der Divinylether zu (2E)-C₆- und/oder (2E)-C₉-Aldehyden;
f) gegebenenfalls die Reduktion der C₆- und/oder C₉-Aldehyde zu C₆- und/oder C₉-Alkoholen;
g) gegebenenfalls die Umsetzung der C₆- und/oder C₉-Alkohole aus Schritt f) mit einer Säure, insbesondere Essigsäure, zu einem entsprechenden Ester.

In einer bevorzugten Ausführungsform wird in dem oben beschriebenen Verfahren zusätzlich ein rekombinantes Nukleinsäuremolekül bereitgestellt, das für ein pflanzliches oder mikrobielles Protein mit der biologischen Aktivität einer 9- und/oder 13-Lipoxygenase (LOX) kodiert. Dies ist insbesondere dann erforderlich, wenn in den eukaryontischen oder prokaryontischen Zellen keine oder nur eine geringe 9- bzw. 13-LOX-Aktivität vorhanden ist.

Nukleotidsequenzen, die für geeignete LOX-Enzyme kodieren, können der Literatur entnommen werden. Bevorzugt werden eine 13-LOX aus Sojabohne (Accession Number: P08170) und/oder eine 9-LOX aus Kartoffel (Accession Number: AAB31252) verwendet.

Unter einer "Wirtszelle" wird erfindungsgemäß jede prokaryotische oder eukaryotische Zelle verstanden, die die erfindungsgemäße 9/13-DES rekombinant exprimiert.

Das vorstehend genannte erfindungsgemäße Verfahren bietet den Vorteil, dass durch die Umsetzung der 9- bzw. 13-Hydroperoxide von Fettsäuren, die Doppelbindungen an den Positionen Δ9 und Δ12 aufweisen, mit 9/13-Divinylethersynthase und die anschließende Umsetzung der Divinylether ausschließlich das stabile (2*E*)-Isomer des gebildeten Aldehyds entsteht. Somit werden nachteilige Produktgemische vermieden, und die gemäß dem erfindungsgemäßen Verfahren erzeugten Aldehyde können mit hoher Reinheit zu (2*E*)-C₆- und/oder C₉-Alkoholen bzw. zu entsprechenden Estern weiterverarbeitet und als Bestandteile von Duft- und Aromastoffen verwendet werden.

Bei der vorliegenden Erfindung werden als mehrfach ungesättigte Fettsäuren C₁₈-Fettsäuren eingesetzt. Dabei können alle C₁₈-Fettsäuren mit einem (9Z, 12Z)-Pentadiensystem umgesetzt werden. Beispiele für C₁₈-Fettsäuren, die vorteilhaft bei dem erfindungsgemäßen Verfahren verwendet werden können, sind α-Linolensäure und Linolsäure. Es können auch Mischungen verschiedener ungesättigter Fettsäuren eingesetzt werden.

Für den Fall, dass die vorstehend genannten mehrfach ungesättigten Fettsäuren, insbesondere α-Linolensäure oder Linolsäure, in der Wirtszelle nicht in ausreichenden Konzentrationen vorliegen, umfasst das erfindungsgemäße Verfahren in einer weiteren Ausführungsform zusätzlich die Bereitstellung zusätzlicher Ausgangssubstrate in der Wirtszelle, z.B. durch exogene Zugabe der Ausgangssubstrate.

Vorzugsweise werden im Anschluss an die Übertragung der Nukleinsäuremoleküle auf pflanzliche Zellen aus den transgenen Zellen vollständige Pflanzen regeneriert, die als Produktionsstätte für Divinylether dienen, die anschließend insbesondere durch mildes Ansäuern bei der Aufarbeitung zu den entsprechenden (2E)-C₆- und/oder C₉-Aldehyden bzw. -Alkoholen bzw. Estern der Alkohole umgesetzt werden.

Bei einer alternativen Ausführungsform der vorliegenden Erfindung handelt es sich bei den transformierten Zellen um Bakterien-, Hefe- oder Algenzellen. In diesem Fall erfolgt die Kultivierung der Zellen und die Herstellung der C₆- und/oder C₉-Aldehyde bzw. -Alkohole bzw. Ester der Alkohole in einem Reaktor.

Bei einer weiteren Ausführungsform werden transgene Pflanzenzellen kultiviert (z.B. in Form von Suspensions- oder Kalluskulturen) und als Produktionsstätte für Divinylether genutzt, die anschließend wiederum wie vorstehend beschrieben zu den entsprechenden C₆- und/oder C₉₋Aldehyden bzw. -Alkoholen bzw. Estern der Alkohole umgesetzt werden.

Vorzugsweise erfolgt die Umsetzung der Divinylether zu den entsprechenden C₆- und/oder C₉-Aldehyden in Schritt e) durch mildes Ansäuern, beispielsweise mit 20%iger HCl.

Je nach Wunsch kann der entstandene Aldehyd in Schritt f) des erfindungsgemäßen Verfahrens enzymatisch, vorzugsweise mit Alkoholdehydrogenase, oder chemisch, vorzugsweise mit Natriumborhydrid, zu einem C₆- und/oder C₉-Alkohol reduziert werden.

Quellen für eine für die Zwecke der vorliegenden Erfindung geeignete Alkoholdehydrogenase sind vorzugsweise Hefen. Die Alkoholdehydrogenase katalysiert die Umwandlung eines Aldehyds in einen Alkohol. Hefe stellt ferner eine Quelle für Nicotinadenindinucleotid (NADH) als Reduktionsmittel bereit.

Alternativ zu der vorstehend beschriebenen Expression der rekombinanten 9/13-Divinylethersynthase und ggf. der 9- bzw. 13-Lipoxygenase in Zellen können die C₆- und/oder C₉-Aldehyde bzw. -Alkohole bzw. Ester der Alkohole auch *in vitro* in einem Reaktionsgemisch hergestellt werden, das als Komponenten eine mehrfach ungesättigte Fettsäure mit Doppelbindungen zumindest an den Positionen Δ9 und Δ12, insbesondere eine C₁₈-Fettsäure, besonders bevorzugt α-Linolensäure oder Linolsäure; 9- und/oder 13-Lipoxygenase und die erfindungsgemäße 9/13-Divinylethersynthase (als Rohextrakt oder in reiner Form); sowie, falls die Herstellung eines Alkohols erwünscht ist, ein Reduktionsmittel; sowie, falls die Herstellung eines Esters erwünscht ist, eine Säure, insbesondere Essigsäure; umfasst.

Ein weiterer Aspekt der vorliegenden Erfindung betrifft Verfahren zur Veränderung des Gehalts an für die Herstellung von C₆- und/oder C₉-Aldehyden bzw. -Alkoholen bzw. Estern der Alkohole als Vorstufe einsetzbaren Divinylether in einer Wirtszelle. Im allgemeinen umfassen die Verfahren das Erhöhen des Gehalts an Divinylethern in einer Wirtszelle. Das Verfahren umfasst die Verwendung von Expressionskonstrukten zur Durchführung der Expression von DNA-Sequenzen, die ein Protein mit der biologischen Aktivität einer 9/13-Divinylethersynthase kodieren, in einer Wirtszelle. Insbesondere bevorzugt ist die Verwendung von Expressionskonstrukten zur Veränderung des Gehalts an Divinylethern als Vorstufe für C₆- und/oder C₉-Aldehyde bzw. -Alkohole bzw. Ester der Alkohole in einer Pflanzenzelle bzw. Pflanze. Vorzugsweise wird das erfindungsgemäße Verfahren zur Veränderung des Gehaltes an Divinylethern in Pflanzenteilen einschließlich Blättern, Wurzeln, Stämmen, Blüten, Früchten, Samen und Saatölen, die aus Pflanzensamen erhalten werden, verwendet.

Besonders bevorzugt werden die für die 9/13-Divinylethersynthase kodierenden DNA-Sequenzen zur Erzeugung von transgenen Pflanzen verwendet, die eine erhöhte Produktion an Divinylethern in Pflanzenfrüchten und -geweben aufweisen. Diese Divinylether können wiederum beispielsweise durch mildes Ansäuern bei der Aufarbeitung in C₆- und/oder C₉₋Aldehyde bzw. -Alkohole bzw. Ester der Alkohole umgesetzt werden. Derartige Aldehyde sind, wie oben beschrieben, wichtige Bestandteile charakteristischer Aromata von Früchten, Gemüse und grünen Blättern. Somit kann die erfindungsgemäße 9/13-Divinylethersynthase auch zur Herstellung von Produkten aus transgenen Pflanzen mit verbesserten Aromamerkmalen verwendet werden.

Um die Lipidperoxidation in einem Pflanzengewebe zu erhöhen, schließt die vorliegende Erfindung auch die Coexpression einer pflanzlichen oder anderen 9/13-Divinylethersynthase in einem Pflanzengewebe mit einem zweiten Gen ein, das bei der Lipidperoxidation eine Rolle spielt. Beispielsweise kann die Coexpression einer 9/13-Divinylethersynthase in einem Pflanzengewebe mit einer DNA-Sequenz, die für eine Lipoxygenase, insbesondere eine 13-Lipoxygenase kodiert, die Lipidperoxidation erhöhen und somit den Gehalt an Divinylethern, die in dem Pflanzengewebe erzeugt werden, steigern. Eine derartige Erhöhung des Gehalts an Divinylethern, die anschließend in C₆- und/oder C₉-Aldehyde umgesetzt werden, kann das "Melonen"- bzw. "Gurken"-Aroma in einem pflanzlichen Produkt erhöhen.

Ferner können die Pflanzenzellen, die eine DNA-Sequenz enthalten, die für eine 9/13-Divinylethersynthase kodiert, ebenfalls als Quelle für Divinylether in Reaktionen für die Erzeugung von C₆- und/oder C₉-Aldehyden bzw. -Alkoholen für die Verwendung in Aromastoffen verwendet werden. Derartige Verfahren sind im Stand der Technik bekannt und sind beispielsweise in der US-Patentschrift 5,695,973 und in der internationalen Patentanmeldung WO 95/26413 beschrieben. Im allgemeinen wird durch derartige Verfahren eine Mischung aus Aldehyden und Alkoholen erhalten. Die Verfahren werden üblicherweise in einem Reaktionsgemisch durchgeführt, das mindestens eine mehrfach ungesättigte Fettsäure, ein Pflanzenmaterial mit einem relativ hohen Gehalt an Enzymaktivität von Lipoxygenase und Divinylethersynthase und eine Alkoholdehydrogenase enthält.

Ein weiterer Gegenstand der Erfindung ist ein Verfahren zur Herstellung von transgenen Pflanzenzellen bzw. transgenen Pflanzen mit erhöhter Pathogenresistenz, umfassend die folgenden Schritte:
a) Herstellen eines rekombinanten Nukleinsäuremoleküls, das folgende Sequenzen umfasst:
   - regulatorische Sequenzen eines in Pflanzenzellen aktiven Promotors;
   - operativ daran gebunden eine Nukleinsäuresequenz, die für ein Protein mit der Aktivität einer 9/13-DES kodiert; und
   - optional operativ daran gebunden regulatorische Sequenzen, die als Transkriptions-, Terminations- und/oder Polyadenylierungssignale in Pflanzenzellen dienen können;
b) Übertragen des Nukleinsäuremoleküls aus a) auf pflanzliche Zellen; und
c) gegebenenfalls die Regeneration von Pflanzen aus den transformierten Pflanzenzellen.

"Transgen" bzw. "rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassetten oder Vektoren alle solche durch gentechnische Methoden zustande gekonimenen Konstruktionen, in denen sich entweder
a) die erfindungsgemäße Nukleinsäuresequenz, oder
b) eine mit der erfindungsgemäßen Nukleinsäuresequenz funktionell verknüpfte genetische Kontrollsequenz, zum Beispiel ein Promotor, oder
c) a) und b)

nicht in ihrer natürlichen, genetischen Umgebung befindet oder durch gentechnische Methoden modifiziert wurde, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der 9/13-DES mit dem entsprechenden 9/13-DES-Gen - wird zu einer transgenen Expressionskassette, wenn diese durch nicht-natürliche, synthetische ("künstliche") Verfahren wie beispielsweise eine Mutagenisierung geändert wird. Entsprechende Verfahren sind beispielsweise beschrieben in US 5,565,350 oder WO 00/15815.

Unter einer transgenen Pflanze bzw. Pflanzenzelle im Sinne der Erfindung ist wie oben ausgeführt zu verstehen, dass sich die im Verfahren verwendeten Nukleinsäuren nicht an ihrer natürlichen Stelle im Genom der Pflanze bzw. Pflanzenzelle befinden, wobei die Nukleinsäuren homolog oder heterolog exprimiert werden können. Transgen bedeutet aber auch, dass die erfindungsgemäßen Nukleinsäuren zwar an ihrem natürlichen Platz im Genom eines Organismus sind, dass jedoch die Sequenz gegenüber der natürlichen Sequenz verändert wurde und/oder dass die Regulationssequenzen der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor.

Wie hier verwendet, betrifft der Begriff "Vektor" ein Nukleinsäuremolekül, das eine andere Nukleinsäure, an welche es gebunden ist, in eine Zelle transportieren kann. Ein Vektortyp ist ein "Plasmid", was für eine zirkuläre doppelsträngige DNA-Schleife darstellt, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch auch andere Expressionsvektorformen, wie virale Vektoren, die ähnliche Funktionen ausüben, umfassen. Ferner soll der Begriff "Vektor" auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Für den Fachmann ist klar, dass die für die Herstellung der transgenen Pflanze bzw. Pflanzenzelle verwendete Nukleinsäuresequenz, die für eine 9/13-DES kodiert, ggf. an die Organismus-spezifische Kodonverwendung angepasst werden muss. Die Kodonverwendung lässt sich anhand von Computerauswertungen anderer bekannter Gene des gewählten Organismus bestimmen.

Bei einem bevorzugten erfindungsgemäßen Verfahren zur Herstellung von transgenen Pflanzen bzw. Pflanzenzellen mit erhöhter Pathogenresistenz wird eine Nukleinsäuresequenz, die für eine erfindungsgemäße 9/13-DES kodiert, auf eine Pflanze bzw. Pflanzenzelle übertragen. Diese Übertragung führt zu einer Erhöhung der Expression bzw. der Aktivität der 9/13-DES im Vergleich zur Wildtyppflanze bzw. -pflanzenzelle und entsprechend zu einer Erhöhung der Pathogenresistenz in den transgenen Pflanzen bzw. Pflanzenzellen.

Die im Verfahren verwendeten Nukleinsäuren können nach Einbringung in eine Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Expression der 9/13-DES durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist.

Unter Erhöhung der Genexpression einer Nukleinsäure, die für eine 9/13-DES kodiert, wird erfindungsgemäß auch die Manipulation der Expression der Pflanzen-eigenen endogenen 9/13-DES verstanden. Dies kann beispielsweise durch Veränderung der Promotor-DNA-Sequenz von Genen, die für die 9/13-DES kodieren, erreicht werden. Eine solche Veränderung, die eine veränderte, vorzugsweise erhöhte Expressionsrate des endogenen 9/13-DES-Gens zur Folge hat, kann durch Deletion oder Insertion von DNA-Sequenzen erfolgen. Eine Veränderung der Promotor-Sequenz von endogenen 9/13-DES-Genen führt in der Regel zu einer Veränderung der exprimierten Menge des 9/13-DES-Gens und damit auch zu einer Änderung der in der Zelle bzw. den Pflanzen nachweisbaren 9/13-DES-Aktivität.

Eine weitere Möglichkeit zur Erhöhung der Aktivität und des Gehalts der endogenen 9/13-DES besteht darin, Transkriptionsfaktoren, die in die Transkription der endogenen 9/13-DES-Gene involviert sind, z.B. durch Überexpression hochzuregulieren. Die Maßnahmen zur Überexpression von Transkriptionsfaktoren sind dem Fachmann bekannt und werden ebenfalls im Rahmen der vorliegenden Erfindung für 9/13-DES offenbart.

Des Weiteren kann eine erhöhte Expression eines endogenen 9/13-DES-Gens dadurch erzielt werden, dass ein im nicht transformierten Organismus nicht vorkommendes Regulatorprotein mit dem Promotor dieser Gene in Wechselwirkung tritt. Solch ein Regulator kann ein chimäres Protein darstellen, welches aus einer DNA-Bindedomäne und einer Transkriptionsaktivator-Domäne besteht, wie beispielsweise in WO 96/06166 beschrieben.

Die rekombinanten Nukleinsäuremoleküle, die zur Expression der 9/13-DES verwendet werden, umfassen neben der zu übertragenden Nukleinsäuresequenz für die 9/13-DES weitere regulatorische Elemente. Welche konkreten regulatorischen Elemente diese Vektoren enthalten müssen, hängt dabei jeweils von dem Verfahren ab, in dem diese Vektoren verwendet werden sollen. Über welche regulatorischen Elemente und auch andere Elemente diese Vektoren verfügen müssen, ist dem Fachmann, der mit den verschiedenen oben erwähnten Verfahren zum Herstellen von transgenen Pflanzen, in denen die Expression eines Proteins gesteigert wird, vertraut ist, bekannt.

Typischerweise handelt es sich bei den regulatorischen Elementen, die in den Vektoren enthalten sind, um solche, die die Transkription und, falls erwünscht, Translation in der Pflanzenzelle gewährleisten. Dies kann beispielsweise je nach ausgewählter Pflanze bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen, an die Induktoren oder Repressoren binden und so die Expression der Nukleinsäure regulieren. Zusätzlich zu diesen neuen Regulationssequenzen oder anstelle dieser Sequenzen kann die natürliche Regulation der Sequenzen vor den eigentlichen Strukturgenen noch vorhanden sein und ggf. genetisch verändert worden sein, so dass die natürliche Regulation ausgeschaltet und die Expression der Gene erhöht wird. Das rekombinante Nukleinsäuremolekül kann aber auch einfacher aufgebaut sein, d.h. es sind keine zusätzlichen Regulationssignale vor die Nukleinsäuresequenz inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen auch allein vor das natürliche Gen zur Steigerung der Aktivität gebracht werden. Das Genkonstrukt kann außerdem vorteilhafterweise auch eine oder mehrere so genannte "Enhancer"-Sequenzen funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren.

Es ist im Prinzip möglich, alle natürlichen Promotoren mit ihren Regulationssequenzen, für das erfindungsgemäße Verfahren zu verwenden. Es ist jedoch auch möglich und vorteilhaft, zusätzlich oder alleine synthetische Promotoren zu verwenden.

Bei den Promotoren kann es sich sowohl um konstitutive, als auch um induzierbare oder gewebe- bzw. entwicklungsspezifische Promotoren handeln. Die Auswahl des Promotors wie auch anderer regulatorischer Sequenzen bestimmt dabei das räumliche und zeitliche Expressionsmuster und damit die Expresson der 9/13-DES in transgenen Pflanzen.

Konstitutive Promotoren schließen ein z.B. den Kern-Promotor des Rsyn7-Promotors und andere konstitutive Promotoren, die in WO 99/43838 und US-Patent Nr. 6,072,050 beschrieben sind; den CaMV 35S- (Odell et al. (1985) Nature 313: 810 - 812); den Actin-(McElroy et al. (1990) Plant Cell 2:163 - 171); den Ubiquitin- (Christensen et al. (1989) Plant Mol. Biol. 12: 619 - 632 und Christensen et al. (1992) Plant Mol. Biol. 18: 675 - 689); den pEMU- (Last et al. (1991) Theor. Appl. Genet. 81: 581 - 588); den MAS- (Velten et al. (1984) EMBO J. 3: 2723 - 2730); den ALS-Promotor (US-Anmeldung Nr. 08/409,297) und ähnliche. Bei Verwendung eines konstitutiven Promotors kann eine zell- bzw. gewebespezifische Expression auch dadurch erreicht werden, dass die Genexpression in den Zellen bzw. Geweben, in denen sie nicht erwünscht ist, gehemmt wird, beispielsweise durch Ausprägung von Antikörpern, die das Genprodukt binden und somit seine Aktivität unterbinden, oder durch geeignete Inhibitoren, die in diesen Zellen wirken.

Bevorzugt wird das 9/13-DES-Gen von einem induzierbaren Promotor exprimiert, und besonders bevorzugt von einem Pathogen-induzierbaren Promotor. Solche Promotoren schließen die von Pathogen-verwandten Proteinen (PR-Proteinen) ein, die nach der Infektion mit einem Pathogen induziert werden, z.B. PR-Proteine, SAR-Proteine, beta-1,3-Glucanase, Chitinase, usw. (siehe z.B. Redolfi et al. (1983) Neth. J. Plant Pathol. 89: 245 - 254; Uknes et al. (1992) Plant Cell 4: 645 - 656; und Van Loon (1985) Plant Mol. Virol. 4: 111 - 116).

Von besonderem Interesse sind auch Promotoren, die örtlich an oder nahe der Stelle der Pathogeninfektion exprimiert werden, wie z.B. die von Marineau et al. (1987) Plant Mol. Biol. 9: 335 - 342; Matton et al. (1989) Molecular Plant-Microbe Interactions 2: 325 - 331; Somsisch et al. (1986) Proc. Natl. Acad. Sci. USA 83: 2427 - 2430; Somsisch et al. (1988) Mol. Gen. Genet. 2: 93 - 98; Yang (1996) Proc. Natl. Acad. Sci. USA 93: 14972 -14977; Chen et al. (1996) Plant J. 10: 955 - 966; Zhang et al. (1994) Proc. Natl. Acad. Sci. USA 91: 2507 - 2511; Warner et al. (1993) Plant J. 3: 191 - 201; Siebertz et al. (1989) Plant Cell 1: 961 - 968 beschriebenen.

Außerdem sind auch wundinduzierbare Promotoren zur Verwendung im Verfahren der vorliegenden Erfindung bevorzugt, da Pathogene häufig durch Wunden eintreten. Solche wundinduzierbaren Promotoren schließen ein den des Proteinase-Inhibitors (pin II) aus Kartoffel (Ryan (1990) Ann. Rev. Phytopathol. 28: 425 - 449; Duan et al. (1996) Nature Biotechnology 14: 494 - 498); wun1 und wun2 (US-Patent Nr. 5,428,148); win1 und win2 (Stanford et al. (1989) Mol. Gen. Genet. 215: 200 - 208); Systemin (McGurl et al. (1992) Science 225: 1570 -1573); WIP1 (Rohmeier et al. (1993) Plant Mol. Biol. 22: 783 - 792; Eckelkamp et al. (1993) FEBS Letters 323: 73 - 76); des MPI-Gens (Corderok et al. (1994) Plant J. 6 (2): 141 - 150); der FGAM-Synthase (Vaghchhipawala et al. (2004) Genome 47 (2): 404 - 413; prxC2 (Kaothien et al. (2002) Plant Mol. Biol. 49 (6): 591 - 599); poxA (Ito et al. (2000) Plant Science 155 (1): 85 - 100); FAD7 (Nishiuchi et al. (1999) Plant Physiol. 121 (4): 1239 - 1246); TR2' (WO 03/093483).

Chemisch regulierte Promotoren können verwendet werden, um die Expression eines Gens in einer Pflanze durch die Anwendung eines exogenen chemischen Regulators zu regulieren (siehe eine Übersicht in Gatz (1997) Annu. Rev. Plant Physiol. Plant Mol. Biol. 48: 89-108). Chemisch induzierbare Promotoren eignen sich besonders, wenn gewünscht wird, dass die Genexpression auf zeitspezifische Weise erfolgt. Beispiele dafür schließen ein den In2-2-Promotor aus Mais, der durch Benzolsulfonamid aktiviert wird, den GST-Promotor aus Mais, der durch hydrophobe elektrophile Verbindungen induziert wird, und den PR-1a-Promotor aus Tabak, der durch Salicylsäure aktiviert wird. Andere chemisch regulierbare Promotoren schließen ein Steroid-responsive Promotoren (siehe z.B. den Glucocorticoid-induzierbaren Promotor in Schena et al. (1991) Proc. Natl. Acad. Sci. USA 88: 10421 -10425 und McNellis et al. (1998) Plant J. 14 (2): 247 - 257), Ethanol-induzierbare und Tetracyclin-induzierbare Promotoren (siehe z.B. Gatz et al. (1991) Mol. Gen. Genet. 227: 229 - 237; US-Patent Nrn. 5,814,618 und 5,789,156).

Der Fachmann weiß, dass die Verwendung von induzierbaren Promotoren die Herstellung von Pflanzen bzw. Pflanzenzellen erlaubt, die die erfindungsgemäßen Sequenzen nur transient exprimieren. Eine solche transiente Expression erlaubt die Herstellung von Pflanzen, die eine nur transiente Pathogenresistenz zeigen. Solch eine transiente Resistenz kann z.B. dann erwünscht sein, wenn die Gefahr einer Pathogenkontamination droht und die Pflanzen deswegen nur für einen bestimmten Zeitraum resistent gegen das Pathogen sein müssen. Weitere Situationen, in denen eine transiente Resistenz wünschenswert ist, sind dem Fachmann bekannt. Dem Fachmann ist darüber hinaus auch bekannt, dass er auch durch die Verwendung von nicht stabil in Pflanzenzellen replizierenden Vektoren, die die entsprechenden Sequenzen zur Expression der 9/13-DES tragen, eine transiente Expression und damit eine transiente Resistenz erzielen kann.

Außerdem können auch Gewebe-spezifische Promotoren verwendet werden, um eine erhöhte 9/13-DES-Expression innerhalb eines bestimmten Pflanzengewebes zu erzielen. Geeignete Gewebe-spezifische Promotoren sind z.B. solche, die Blatt-spezifische Expression gewährleisten. Zu diesen gehören solche, die in Yamamoto et al. (1997) Plant J. 1 (2): 255 - 265; Kwon et al. (1994) Plant Physiol 105: 357-367; Yamamoto et al. (1994) Plant Cell Physiol. 35 (5): 773-778; Gotor et al. (1993) Plant J. 3: 509-518; Orozco et al. (1993) Plant Mol. Biol. 23 (6): 1129-1138: Matsuoka et al. (1993) Proc. Natl. Acad. Sci. USA 90 (20): 9586-9590; Stockhaus et al. (1987) Proc. Natl. Acad. G. USA 84: 7943 - 7947; Stockhaus et al. (1989) EMBO J. 8: 2445 - 2451 beschrieben sind.

Insbesondere bevorzugt ist es auch, Epidermis-spezifische Promotoren zu verwenden, da die Epidermis als Abschlussgewebe der oberirdischen Organe höherer Pflanzen unter anderem die Aufgabe hat, das Eindringen von Pathogenen in die Pflanze zu verhindern. Zu den geeigneten epidermis-spezifischen Promotoren gehört u.a. der Promotor des CER6- (CUT1-) Gens aus *Arabidopsis* (Hooker et al. (2002) Plant Physiol. 129 (4): 1568 -1580 und Kunst et al. (2000) Biochem. Soc. Trans. 28 (6): 651 - 654).

Weiterhin bevorzugte Promotoren sind solche, die insbesondere in Früchten aktiv sind. Hierzu zählen beispielsweise der Promotor eines Polygalacturonase-Gens, z.B. aus Tomate (Nicholass *et al.* (1995) Plant Mol. Biol. 28:423-435), der Promotor einer ACC-Oxidase, z.B. aus Apfel (Atkinson *et al.* (1998) Plant Mol. Biol. 38:449-460) oder der 2A11-Promotor aus Tomate (van Haaren *et al*. (1991) Plant Mol. Biol. 17:615-630).

Als samenspezifische Promotoren bieten sich bspw. der USP- und Vicilin-Promotor (Bäumlein et al. (1991), Mol. Gen. Genet. 225: 459-467), der Hordein-Promotor (Brandt et al. (1985), Carlsberg Res. Commun. 50: 333-345), der Napin- (US 5,608,152), Conlinin-(WO 02/102970),der Acyl-Carrier Protein- (US 5,315,001 und WO 92/18634), der Oleosin-(WO 98/45461 und WO 93/20216), der Phaseolin- (US 5,504,200), Bce4- (WO 91/13980), der LegB4- (Bäumlein et al., Plant J., 2,2, 1992), der Lpt2- und lpt1-Promotor (WO 95/15389 und WO95/23230), Samen-spezifische Promotoren aus Reis, Mais u. Weizen [WO 99/16890], der Amy32b-, Amy 6-6- und Aleurain- (US 5,677,474), der Bce4- (US 5,530,149), der Glycinin- (EP 571 741), der Phosphoenol-Pyruvatcarboxylase (JP 06/62870), der ADR12-2-(WO 98/08962), der Isocitratlyase- (US 5,689,040) oder der α-Amylase-Promotor (EP 781 849).

Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z.B. Hybridisierungsexperimenten oder DNA-Protein-Bindungsstudien z. B. weitere samen-spezifische regulatorische Nukleinsäureelemente identifizieren. Dazu wird z.B. in einem ersten Schritt das gewünschte Gewebe, z.B. der Samen, aus dem gewünschten Organismus, aus dem die regulatorischen Sequenzen isoliert werden sollen, isoliert und daraus die gesamte poly(A)⁺-RNA isoliert und eine cDNA-Bank angelegt. In einem zweiten Schritt werden mit Hilfe von cDNA-Klonen, die auf poly(A)⁺⁻RNA-Molekülen aus einem anderen Gewebe basieren, aus der ersten Bank mittels Hybridisierung diejenigen Klone identifiziert, deren korrespondierende poly(A)⁺-RNA-Moleküle lediglich im gewünschten Gewebe akkumulieren. Anschließend werden mit Hilfe dieser so identifizierten cDNAs Promotoren isoliert, die über Gewebe-spezifische regulatorische Elemente verfügen. Dem Fachmann stehen darüber hinaus weitere auf PCR basierende Methoden für die Isolierung geeigneter Gewebe-spezifischer Promotoren zur Verfügung.

Die erfindungsgemäßen Vektoren können als regulatorische Elemente zusätzlich auch z.B. Enhancer-Elemente umfassen, ferner können sie Resistenzgene, Replikationssignale und weitere DNA-Bereiche enthalten, die eine Propagation der Vektoren in Bakterien wie z.B. *E. coli* ermöglichen. Die regulatorischen Elemente umfassen auch Sequenzen, die eine Stabilisierung der Vektoren in den Wirtszellen bewirken. Insbesondere umfassen solche regulatorischen Elemente Sequenzen, die eine stabile Integration des Vektors in das Wirtsgenom der Pflanze oder eine autonome Replikation des Vektors in den Pflanzenzellen ermöglichen. Solche regulatorischen Elemente sind dem Fachmann bekannt.

Bei den so genannten Terminationssequenzen handelt es sich um Sequenzen, die sicherstellen, dass die Transkription bzw. die Translation ordnungsgemäß beendet wird. Sollen die übertragenen Nukleinsäuren translatiert werden, handelt es sich bei den Terminationssequenzen typischerweise um Stopcodons und entsprechende regulatorische Sequenzen; sollen die übertragenen Nukleinsäuren nur transkribiert werden, handelt es sich in der Regel um poly(A)-Sequenzen. Derartige Elemente sind in der Literatur beschrieben (z.B. Gielen (1989) EMBO J. 8:23-29) und beliebig austauschbar, z.B. der Terminator des Octopinsynthasegens aus *Agrobacterium tumefaciens.*

In einem rekombinanten Nukleinsäuremolekül bedeutet "operativ daran gebunden", dass die Nukleotidsequenz von Interesse derart an die Regulationssequenz(en) gebunden ist, dass die Expression der Nukleotidsequenz möglich ist und die beiden Sequenzen derart aneinander gebunden sind, dass sie die vorhergesagte, der Sequenz zugeschriebene Funktion erfüllen.

Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder in Gruber und Crosby, in: Methods in Plant Molecular Biology and Biotechnology, CRC Press, Boca Raton, Florida, Hrsgb.: Glick und Thompson, Kapitel 7, 89-108. Regulationssequenzen umfassen solche, welche die konstitutive Expression einer Nukleotidsequenz in vielen Wirtszelltypen steuern, und solche, die die direkte Expression der Nukleotidsequenz nur in bestimmten Wirtszellen unter bestimmten Bedingungen steuern. Der Fachmann weiß, dass die Gestaltung des Expressionsvektors von Faktoren wie der Auswahl der zu transformierenden Wirtszelle, dem gewünschten Ausmaß der Expression des Proteins usw., abhängen kann.

Die verwendeten rekombinanten Nukleinsäuremoleküle zur Expression der 9/13-DES können sowohl in prokaryotischen als auch in eukaryotischen Zellen aktiv sein. Dies ist vorteilhaft, da häufig Zwischenschritte der Vektorkonstruktion der Einfachheit halber in Mikroorganismen durchgeführt werden und zudem die Herstellung der Divinylether, Aldehyde, Alkohole und Ester der Alkohole mit Hilfe der erfindungsgemäßen 9/13-DES auch in Mikroorganismen stattfinden kann. Geeignete Klonierungsvektoren enthalten ein Replikationssignal für den entsprechenden Mikroorganismus und ein Markergen zur Selektion erfolgreich transformierter Bakterienzellen. Zur Expression in prokaryotischen Organismen geeignete Vektoren sind dem Fachmann bekannt, zu diesen gehören z.B. in *E. coli* pLG338, pACYC184, die pBR-Reihe, wie z.B. pBR322, die pUC-Reihe, wie z.B. pUC 18 oder pUC 19, die M113mp-Reihe, pKC30, pRep4, pHS1, pHS2, pPLc236, pMBL24, pLG200, pUR290, pIN-III113-B1, λgt11 oder pBdCl, in *Streptomyces* pIJ101, pIJ364, pIJ702 oder pIJ361, in *Bacillus* pUB110, pC 194 oder pBD214, in *Corynebacterium* pSA77 oder pAJ667.

Bei einer weiteren Ausführungsform stellt der Expressionsvektor einen Hefeexpressionsvektor oder einen Bacillovirus-Expressionsvektor dar.

Die oben genannten Vektoren bieten nur einen kleinen Überblick über mögliche geeignete Vektoren. Weitere Plasmide sind dem Fachmann bekannt und sind z.B. beschrieben in: Cloning Vectors (Hrsg. Pouwels, P.H. et al. Elsevier, Amsterdam, New York-Oxford, 1985). Weitere geeignete Expressionssysteme für prokaryotische und eukaryotische Zellen siehe in den Kapiteln 15 und 16 von Sambrook und Russell, vide supra.

Bei einer weiteren Ausführungsform des Verfahrens kann die 9/13-DES in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992), Plant Mol. Biol. 20:1195-1197; und Bevan, M.W. (1984), Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Da die Pflanzengenexpression sehr oft nicht auf die Transkriptionsebene beschränkt ist, enthält eine Pflanzen-Expressionskassette vorzugsweise zusätzlich zu den oben beschriebenen Elemente andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al. (1987) Nucl. Acids Research 15:8693-8711).

Das zu exprimierende Gen muss- wie oben beschrieben- funktionsfähig mit einem geeigneten Promotor verbunden sein, der die Genexpression auf rechtzeitige, zell- oder gewebespezifische Weise steuert. Geeignete Promotoren wurden bereits oben beschrieben.

Andere bevorzugte Sequenzen für die Verwendung zur funktionsfähigen Verbindung in Genexpressionskassetten sind Targeting-Sequenzen, die zur Steuerung des Genproduktes in das entsprechende Zellkompartiment notwendig sind (siehe eine Übersicht in Kermode (1996) Crit. Rev. Plant Sci. 15 (4): 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Zum Einbringen der 9/13-DES-Sequenzen in die rekombinanten Nukleinsäuremoleküle werden diese vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Bakterien, Hefen oder Pilzen gewährleisten, und die die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in *E*. *coli* als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al. (2000) Trends in Plant Science 5,446-451.

Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung werden das enzymatisch geschnittene und erforderlichenfalls gereinigte Amplifikat mit ähnlich präparierten Vektorfragmenten durch eine Ligase miteinander verknüpft. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere *E. coli* und *Agrobacterium tumefaciens*, in einem geeigneten Medium züchten und unter Selektionsbedingungen stabil propagieren. Anschließend werden die Zellen geerntet und lysiert und das Plasmid daraus gewonnen. Dies ermöglicht einen Transfer von heterologer DNA in Pflanzen oder Mikroorganismen.

Unter der vorteilhaften Verwendung von Klonierungsvektoren können die im erfindungsgemäßen Verfahren verwendeten Nukleinsäuren, die erfinderischen Nukleinsäuren und Nukleinsäurekonstrukte in Organismen wie Mikroorganismen oder bevorzugt Pflanzen eingebracht werden und zur Pflanzentransformation verwendet werden, wie diejenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus (1991) Annu. Rev. Plant Physiol. Plant Molec. Biol. 42: 205-225. Die im Verfahren verwendeten Nukleinsäuren, die erfinderischen Nukleinsäuren und Nukleinsäurekonstrukte und/oder Vektoren lassen sich damit zur gentechnologischen Veränderung eines breiten Spektrums an Organismen, bevorzugt von Pflanzen, verwenden.

Für die Einführung von DNA in eine pflanzliche Wirtszelle stehen eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichkeiten. Dabei können sowohl stabile als auch transiente Transformanten erzeugt werden.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide wie z.B. pUC-Derivate verwendet werden. Sollen aber aus derartig transformierten Zellen ganze Pflanzen regeneriert werden, ist die Anwesenheit eines selektierbaren Markergens notwendig. Dem Fachmann sind die gängigen Selektionsmarker bekannt und es stellt für ihn kein Problem dar, einen geeigneten Marker auszuwählen. Gebräuchliche Seklektionsmarker sind solche, die den transformierten Pflanzenzellen Resistenz gegenüber einem Biozid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin und dergleichen vermitteln. Der individuell gewählte Marker sollte die Selektion transformierter Zellen gegenüber Zellen, denen die eingeführte DNA fehlt, gestatten. Hierzu sind auch alternative Marker geeignet, wie nutritive Marker oder Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls markerfreie transgene Pflanzen erwünscht sind, stehen dem Fachmann auch Strategien zur Verfügung, die eine nachträgliche Entfernung des Markergens erlauben, z. B. Cotransformation oder Sequenz-spezifische Rekombinasen. Ist die eingeführte DNA einmal im Genom der Pflanzenzelle integriert, so ist sie dort in der Regel stabil und bleibt auch in den Nachkommen der ursprünglich transformierten Zelle erhalten.

Werden für die Transformationen Agrobakterien verwendet, muss die einzuführende DNA, wie oben bereits ausgeführt, in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri-Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf Agrobacterium tumefaciens übertragen werden (Konjugation). Binäre Vektoren können sowohl in E. coli als auch in Agrobakterien replizieren. Sie enthalten ein Selektionsmarker-Gen und einen Linker oder Polylinker, welche von der rechten und linken T-DNA-Grenzregion eingerahmt werden. Sie können direkt in die Agrobakterien transformiert werden (Holsters et al. (1978), Molecular and General Genetics 163, 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzlich kann T-DNA vorhanden sein. Das derartig transformierte Agrobakterium wird zur Transformation von Pflanzenzellen verwendet.

Die Verwendung von T-DNA für die Transformation von Pflanzenzellen ist intensiv untersucht und ausreichend in EP 120 515 beschrieben worden.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (beispielsweise Blattstücke, Stengelsegmente, Wurzeln, aber auch Protoplasten oder Suspensions-kultivierte Pflanzenzellen) können dann in einem geeignetem Medium, welches Antibiotika oder Biozide zur Selektion transformierter Zellen enthalten kann, wieder ganze Pflanzen regeneriert werden. Die Regeneration der Pflanzen erfolgt nach üblichen Regenerationsmethoden unter Verwendung bekannter Nährmedien. Die so erhaltenen Pflanzen bzw. Pflanzenzellen können dann auf Anwesenheit der eingeführten DNA mit etablierten Methoden wie z.B. Southern Blot oder PCR untersucht werden.

Andere Möglichkeiten der Einführung fremder DNA unter Verwendung des biolistischen Verfahrens oder durch Protoplasten-Transformation sind dem Fachmann bekannt (vgl. L. Willmitzer (1993) Transgenic Plants in: Biotechnology, A Multi-Volume Comprehensive Treatise (Herausgeber: H.J. Rehm et al.), Band 2, 627-659, VCH Weinheim, Deutschland).

Inzwischen ist nicht nur die Transformation dikotyler Pflanzen bzw. derer Zellen über Ti-Plasmid-Vektorsysteme mit Hilfe von Agrobacterium tumefaciens gut etabliert, sondern auch die monokotyler Pflanzen bzw. derer Zellen (siehe u.a. Chan et al. (1993), Plant Mol. Biol. 22, 491-506).

Alternative Systeme zur Transformation von monokotylen Pflanzen bzw. deren Zellen sind die Transformation mittels des biolistischen Ansatzes (Wan und Lemaux (1994) Plant Physiol. 104, 37-48; Vasil et al. (1993) Bio/Technology 11, 1553-1558; Ritala et al. (1994) Plant Mol. Biol. 24, 317-325; Spencer et al. (1990), Theor. Appl. Genet. 79, 625-631), die Protoplasten-Transformation, die Elektroporation von partiell permeabilisierten Zellen sowie die Einbringung von DNA mittels Glasfasern.

Die transformierten Zellen wachsen innerhalb der Pflanze in üblicher Weise (siehe auch McCormick et al. (1986), Plant Cell Reports 5, 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen besitzen die entsprechenden phänotypischen Eigenschaften.

Es sollten zwei oder mehrere Generationen angezogen werden, um sicherzustellen, dass das phänotypische Merkmal stabil beibehalten und vererbt wird. Auch sollten Samen geerntet werden, um sicherzustellen, dass der entsprechende Phänotyp oder andere Eigenarten erhalten geblieben sind.

Ebenso können nach üblichen Methoden transgene Linien identifiziert werden, die für die neuen Nukleinsäuremoleküle homozygot sind und ihr phänotypisches Verhalten hinsichtlich einer vorhandenen bzw. nicht vorhandenen Pathogen-Responsivität untersucht und mit dem von hemizygoten Linien verglichen werden.

Selbstverständlich können die Pflanzenzellen, die die erfindungsgemäßen Nukleinsäuremoleküle enthalten, auch in Form einer Zellkultur (einschließlich Protoplasten, Kalli, Suspensionskulturen und dergleichen) weiterkultiviert werden.

Das erfindungsgemäße Verfahren kann vorteilhaft mit weiteren Verfahren, die eine Pathogenresistenz (beispielsweise gegen Insekten, Pilze, Bakterien, Nematoden, usw.), Stressresistenz oder eine andere Verbesserung der pflanzlichen Eigenschaften bewirken, kombiniert werden. Beispiele sind u.a. genannt in Dunwell JM (2000) J Exp Bot. 51: 487-496.

Erfindungsgemäß umfasst der Begriff transgene Pflanze sowohl die Pflanze in ihrer Gesamtheit, als auch alle Pflanzenteile, in denen erfindungsgemäß die Expression der 9/13-DES erhöht ist. Dazu gehören alle Pflanzenteile und Pflanzenorgane wie Blatt, Stiel, Samen, Wurzel, Knollen, Antheren, Fasern, Wurzelhaare, Stengel, Embryos, Kalli, Kotelydonen, Petiolen, Erntematerial, pflanzliches Gewebe, reproduktives Gewebe, Zellkulturen, die sich von der transgenen Pflanze ableiten und/oder dazu verwendet werden können, die transgene Pflanze hervorzubringen.

Je nach dem verwendeten Vektorsystem können erfindungsgemäß auch transgene Pflanzen hergestellt werden, bei denen die zu übertragenden Nukleinsäuren in der Pflanzenzelle bzw. der Pflanze als selbstständig replizierendes System enthalten sind. Die zur Übertragung der Pflanzen verwendeten Vektoren müssen dann entsprechend über DNA-Sequenzen verfügen, die die Replikation von zur Übertragung verwendeten Plasmiden innerhalb der Zelle ermöglichen.

Die spezifische Expression des 9/13-DES-Proteins in den erfindungsgemäßen Pflanzen bzw. in den erfindungsgemäßen Pflanzenzellen kann mit Hilfe herkömmlicher molekularbiologischer und biochemischer Methoden nachgewiesen und verfolgt werden. Dem Fachmann sind diese Techniken bekannt und er ist problemlos in der Lage, eine geeignete Nachweismethode zu wählen, beispielsweise eine Northern-Blot-Analyse zum Nachweis 9/13-DES-spezifischer RNA bzw. zur Bestimmung der Höhe der Akkumulation von 9/13-DES-spezifischer RNA oder eine Southern-Blot- bzw. PCR-Analyse zum Nachweis von für die 9/13-DES kodierenden DNA-Sequenzen. Die dazu verwendeten Sonden- bzw. Primersequenzen können zu der in SEQ ID No. 1 angegebenen Sequenz entweder identisch sein oder einige wenige Abweichungen von dieser Sequenz aufweisen.

Bei den für das erfindungsgemäße Verfahren verwendeten Pflanzen kann es sich im Prinzip um jede beliebige Pflanze handeln, die resistent gegen Pathogenbefall gemacht werden soll. Vorzugsweise ist es eine monokotyle oder dikotyle Nutz-, Nahrungs- oder Futterpflanze.

Beispiele für monokotyle Pflanzen sind Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) und dergleichen gehören.

Dikotyle Nutzpflanzen umfassen unter anderem Baumwolle, Leguminosen wie Hülsenfrüchte und insbesondere Alfalfa, Sojabohne, Raps, Canola, Tomate, Zuckerrübe, Kartoffel, Sonnenblume, Zierpflanzen sowie Bäume. Weitere Nutzpflanzen können Obst (insbesondere Äpfel, Birnen, Kirschen, Weintrauben, Citrus, Ananas und Bananen), Ölpalmen, Tee-, Kakao- und Kaffeesträucher, Tabak, Sisal sowie bei Heilpflanzen Rauwolfia und Digitalis umfassen. Besonders bevorzugt sind die Getreide Weizen, Roggen, Hafer, Gerste, Reis, Mais und Hirse, sowie die dikotylen Pflanzen Zuckerrübe, Raps, Soja, Tomate, Kartoffel und Tabak. Weitere Nutzpflanzen können dem US-Patent Nr. 6,137,030 entnommen werden.

Bevorzugte Pflanzen sind Tagetes, Sonnenblume, Arabidopsis, Tabak, Roter Pfeffer, Soja, Tomate, Aubergine, Paprika, Möhre, Kartoffel, Mais, Salate und Kohlarten, Getreide, Alfalfa, Hafer, Gerste, Roggen, Weizen, Triticale, Hirse, Reis, Luzerne, Flachs, Baumwolle, Hanf, Brassicacaeen wie beispielsweise Raps oder Canola, Zuckerrübe, Zuckerrohr, Nuss- und Weinspezies oder Holzgewächse wie beispielsweise Espe oder Eibe.

Besonders bevorzugte Pflanzen sind Weizen, Gerste, Kartoffel und Ölsaaten.

Bei den "Pathogenen" kann es sich um jeden Erreger handeln, der die Wildtyppflanze normalerweise befällt, also Pilz-, bakterielle, virale und Tierpathogene. Bevorzugt handelt es sich um Pilzpathogene wie beispielsweise *Blumeria graminis* oder *Phytophtora infestans.* Es ist aber auch anzunehmen, dass die Überexpression der erfindungsgemäßen Sequenz auch eine Resistenz gegenüber weiteren Pathogenen wie *Phytium spec., Albugo spec., Rhizoctonia solani, Peronospora parasitica, Erysiphe crucifearum, E. cichoreacearum, Alternaria brassicicola, Botrytis cinerea, Sclerotium rolfsii, Sclerotinia sclerotium, Fusarium oxysporum, F. culmorum, F. graminearum, F. nivale* oder *Pseudomonas syringae* bewirkt.

"Pathogenresistenz" meint das Vermindern oder Abschwächen von Krankheitssymptomen einer Pflanze infolge eines Befalls durch ein Pathogen. Die Symptome können vielfältiger Art sein, umfassen aber bevorzugt solche, die direkt oder indirekt zu einer Beeinträchtigung der Qualität der Pflanze, der Quantität des Ertrags, der Eignung zur Verwendung als Futter- oder Nahrungsmittel führen oder aber auch Aussaat, Anbau, Ernte oder Prozessierung des Ernteguts erschweren.

Unter dem Begriff "erhöhte Pathogenresistenz" wird erfindungsgemäß verstanden, dass die erfindungsgemäßen transgenen Pflanzen bzw. Pflanzenzellen durch Pathogene weniger stark und/oder weniger häufig befallen werden als nicht-transformierte Wildtyp-Pflanzen bzw. Pflanzenzellen, die ansonsten gleich behandelt wurde (z.B. Klima- und Anbaubedingungen, Pathogenart, usw.). Bevorzugt ist der Befall mit Pathogenen mindestens um den Faktor 2, besonders bevorzugt mindestens um den Faktor 3, insbesondere bevorzugt mindestens um den Faktor 5 und am meisten bevorzugt mindestens um den Faktor 10 reduziert, was sich in einer Verminderung der Ausbildung von Krankheitssymptomen äußert. Der Begriff "erhöhte Pathogenresistenz" beinhaltet dabei auch eine so genannte transiente Pathogenresistenz, d.h. dass die erfindungsgemäßen transgenen Pflanzen bzw. Pflanzenzellen nur für einen begrenzten Zeitraum eine gegenüber dem entsprechenden Wildtyp erhöhte Pathogenresistenz aufweisen. Ermittelt werden kann die Pathogenresistenz der Pflanze durch die Abnahme der Penetrationsfrequenz. Unter Penetrationsfrequenz ist erfindungsgemäß die Anzahl der Infektionsstellen mit erfolgreich penetrierten Epidermiszellen zu verstehen, dividiert durch die Anzahl der Infektionsstellen.

Die erfindungsgemäße 9/13-DES kann, wie oben beschrieben, auch dazu verwendet werden, Divinylether *in vitro* herzustellen. Diese Divinylether können dann auf Pflanzen appliziert werden und in ihnen eine Pathogenresistenz hervorrufen. Die Applikation kann beispielsweise durch Sprühen mit hohem oder niedrigem Druck, Injektion oder Einnebeln erfolgen. Damit kann entweder die gesamte Pflanze oder nur ein Teil dieser Pflanzen wie beispielsweise Blätter, Stamm oder Wurzeln behandelt werden. Die Zusammensetzung, die zur Behandlung der Pflanzen verwendet wird, enthält mindestens einen geeigneten Trägerstoff. Geeignete Trägerstoffe schließen zum Beispiel ein Wasser, Lösungsmittel, wässrige Lösungen oder trockene Pulver; zusätzliche Trägerstoffe schließen ein Kieselgur und Petrolatum-Produkte. Eine solche Zusammensetzung kann weitere Komponenten, wie zum Beispiel Düngemittel, Nährstoffe, Hormone, Insektizide, Fungizide, Nematozide und Herbizide enthalten. Weitere geeignete Bestandteile der Zusammensetzung sind dem Fachmann bekannt.

Die nachfolgenden Beispiele dienen der Beschreibung der vorliegenden Erfindung, ohne diese in irgendeiner Weise einzuschränken.

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose- und Nylon-Membranen, Verknüpfen von DNA-Fragmenten, Transformation von E. coli-Zellen, Anzucht von Bakterien und die Sequenzanalyse rekombinanter DNA wurden wie bei Sambrook et al. (2001), vide supra, beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA 74, 5463-5467).

### Beispiel 3: Klonierung der 9/13-DES aus Knoblauch

Von dem EST-Klon EST682106 aus Zwiebel mit einer Länge von 909 Basenpaaren und 27 % Homologie zu einer DES aus Tabak (GP 17646111) wurden folgende Primer für die PCR und RACE-PCR abgeleitet:

CF44_sonder | GCTTCACCAGCTTCTTGCCGTC

CF44_sondeu GGCGTTTACGAAGTTTAACGAGGC

CF44_5race GCCTCGTTAAACTTCGTAAACGCC

CF44_3race GACGGCAAGAAGCTGGTGAAGC

Diese Primer wurden in einer PCR mit cDNA, die aus Knoblauchwurzeln gewonnen wurde, eingesetzt. Die PCR-Bedingungen waren wie folgt: 2 Minuten 95°C; 15 Zyklen 30 Sekunden 95°C, 30 Sekunden bei 50 bis 52°C, und 90 Sekunden bei 72°C; 15 Zyklen 30 Sekunden 95°C, 30 Sekunden 50 bis 52°C, und 90 Sekunden 72°C und eine Verlängerung der Inkubationszeit für 5 Sekunden pro Zyklus; 5 Minuten bei 72°C.

Das so erhaltene cDNA-Fragment aus Knoblauch, das ca. 850 Basenpaare lang war, wurde in den Vektor p-GEM-T (Promega, Madison, USA) kloniert. Um die vollständige kodierende Sequenz zu erhalten, wurde ein 3' RACE-PCR und eine 5' RACE-PCR durchgeführt, für die mit Hilfe des Smart™ RACE cDNA Amplification-Kits (Clontech, Palo Alto, USA) eine RACE-cDNA-Bank hergestellt wurde.

Für die 3'-RACE-PCR wurde, nachdem versucht worden war, das fehlende Ende mit dem 3'-RACE-Primer und dem UMP-Primer aus dem Kit zu amplifizeren, eine Nested-PCR durchgeführt. Als 5'-Primer wurde der CF44_sondeu-Primer verwendet und als 3'-Primer 3 µl des UMP-Primers aus dem Kit. Die PCR wurde mit einem Temperaturprogramm wie oben angegeben durchgeführt, allerdings wurde die Verlängerungszeit auf 30 s verkürzt, die Annealing-Temperatur lag bei 52°C. Als Template für die folgende PCR dienten 2 µl des ersten Ansatzes. Es wurden zwei neue Primer eingesetzt, die weiter innerhalb des gesuchten Fragments lagen, um die Produktspezifität zu erhöhen. Als 3'-Primer wurde der CF44_3race-Primer verwendet und als 5'-Primer der Nested-Primer des Kits.

Für die Amplifizierung der vollständigen cDNA zwecks Klonierung in den Expressionsvektor pQE 30 (Qiagen, Hilden) wurden Primer gewählt, die vor den 5'- und 3'-terminalen Sequenzen der cDNA Restriktionsschnittstellen enthielten:

AsDesBamHIuni GGATCCATGTCCACCTCCAACGGCAGCAC

AsDesPstIrev2 | CTGCAGTCAGACTGCTACCTTGGTCAGCTC

Die Restriktionsenzyme wurden so gewählt, dass sie die cDNA nicht schnitten, wohl aber die Multiple-Cloning-Site des Expressionsvektors. Zur Amplifizierung der vollständigen cDNA-Klone wurde der Expand™ High-Fidelity Kit (Roche Diagnostics, Grenzach) verwendet.

Die so erhaltene vollständige cDNA-Sequenz ist in SEQ ID Nr. 1 gezeigt, die durch diese DNA-Sequenz kodierte erfindungsgemäße 9/13-DES aus Knoblauch ist in SEQ ID Nr. 2 gezeigt.

### Beispiel 4: Expression der rekombinanten 9/13-DES in E. coli

Die Expression des rekombinanten Proteins erfolgte im *E. coli*-Stamm SG13009 [pREP4], der mit dem Expressionsvektor pQE 30, der die 9/13-DES enthielt, transformiert worden war. Zur Expression wurden zunächst 3 ml LB-Medium mit entsprechendem Antibiotikum mit *E*. *coli-*SG13009 [pRET4] mit Expressionsvektor angeimpft und über Nacht bei 37°C und Schütteln inkubiert. Für die Hauptkultur wurden 30 ml Medium mit entsprechendem Antibiotikum (Carbenicillin und Kanamycin) mit dieser Vorkultur angeimpft und bei 37°C inkubiert. Ab einer OD₆₀₀ von 0,6 bis 0,8 erfolgte die Induktion mit IPTG (Endkonzentration 1 mM), anschließend wurden die Bakterien für bis zu 2 Tage bei 16°C kultiviert.

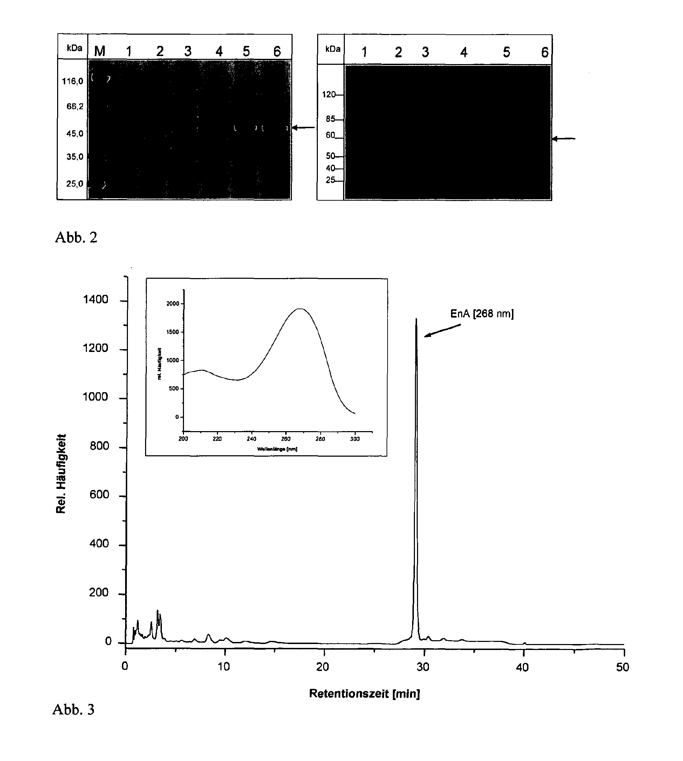

Zur Ernte der *E. coli*-Zellen wurden diese für 15 Minuten bei 4000 g sedimentiert und in 100 mM Natriumphosphatpuffer pH 8,0 aufgenommen, mit Ultraschall (Sonoplus GM70, Bandelin, Berlin) für fünf Mal 1 Minute bei 50% Leistung und 50% Impuls aufgeschlossen. Mit dem Lysat wurde zunächst die Expression des Proteins im Western Blot überprüft (vgl. Abb. 2). Die Durchführung des Western Blots erfolgte nach Standardprotokollen.

### Beispiel 5: Aktivitätstests der rekombinanten Enzyme

Für die Aktivitätstests mussten zunächst die Substrate der 9/13-DES, nämlich die Fettsäurehydroperoxide, hergestellt werden. Dazu wurden zwei verschiedene Lipoxygenasen und zwischen 10 und 20 mg Fettsäure in einem Gesamtvolumen von 10 ml Puffer eingesetzt. Bei den Lipoxygenasen handelte es sich um Soja-LOX Typ V oder um StLOX. Bei den Fettsäuren handelt es sich um Linolsäure oder um α-Linolensäure. Als Puffer wurde entweder Borat pH 10,4 (für Soja-LOX Typ V) oder Phosphat pH 7,5 (für StLOX) verwendet. Der Reaktionsansatz wurde für eine Stunde auf Eis unter Rühren inkubiert. Durch Ansäuern mit Eisessig auf pH 4 wurde die Reaktion gestoppt und anschließend die Hydroperoxide mit 20 ml Methanol/Chloroform extrahiert (Blight und Dyer (1959) Can J Biochem Physiol. 37: 911-917). Es folgte eine Trennung der Phasen für 5 Minuten bei 4°C bei 4000 g, bevor die Chloroformphase mit einer Pasteurpipette in ein 50 ml Reaktionsgefäß überführt wurde. Diese Extraktion wurde zwei Mal wiederholt und die vereinigten Chloroformphasen unter Stickstoffstrom eingedampft und in 5 ml 7% Diethylether in Hexan resuspendiert. Um Eduktreste zu entfernen, wurde der Rückstand über eine selbstgepackte Kieselgelsäule (CC4-Special, Mallinckrodt Specialty Chemicals Co., Paris, Kentucky, USA) gereinigt. Dazu wurden 2 g Kieselgel für 1 h bei 180°C getrocknet und in ca. 30 ml 7% Diethylether/Hexan equilibriert. Das Säulenmaterial wurde in eine Bürette geben und die Probe aufgetragen. Anschließend wurden die Eduktreste mit ca. 60 ml 7% Diethylether/Hexan von der Säule eluiert. Nach volständiger Elution des Edukts wurde das Hydroperoxid mit 20% Diethylether/Hexan eluiert. Die Fraktionen mit der größten Absorption bei der Wellenlänge des Produktes wurden vereinigt und unter Stickstoffstrom eingedampft. Für die Enzymumsetzungen wurde das Pellet je nach eingesetzter Menge in 1 bis 2 ml Methanol gelöst.

Für den Aktivitätstest wurden 1,2 ml Hydroperoxid-Lösung (in 100 mM Natriumphosphatpuffer, pH 6,5) mit 800 µl Zelllysat versetzt und 30 Minuten bei Raumtemperatur inkubiert. Danach wurde mit Eisessig auf pH 4,0 angesäuert und die Produkte mit 4 ml Chloroform/Methanol (1:1 (v/v)) extrahiert. Die organische Phase wurde im Stickstoffstrom bis zur Trockne eingedampft und in 50 µl HPLC-Laufmittel (Laufmittel A) aufgenommen.

### Beispiel 6: Nachweis der gebildeten Divinylether durch Umkehrphasen-HPLC

Die Analysen der Produkte des Aktivitätstests wurden an einem Hewlett Packard HPLC-System, welches einen Dioden-Array-Detektor enthielt (Agilent 1100, Waldbronn), mit den folgenden Parametern durchgeführt:
Säule: 250 x 2 mm, 5 µm Partikelgröße, EC 250/2 NUCLEOSIL 120-5 C-18 von Macherey-Nagel (Düren)
Laufmittel: A: Acetonitril/Wasser/Eisessig (50:50:0,1, v/v/v)
B: Acetonitril/Eisessig (100:0,1, v/v/v)
Injektionsvolumen: 50 µl
Fluss: 1 ml/min
Laufschema:

| Zeit [min] | % Laufmittel B | Fluss [ml/min] |
|---|---|---|
| 0 | 0 | 1 |
| 25 | 0 | 1 |
| 26 | 60 | 1 |
| 36 | 60 | 1 |
| 38-50 | 0 | 1 |

Die Produkte wurden anhand ihrer Absorption bei 268 und 252 nm detektiert. Das Produkt des rekombinanten Enzyms hatte ein Absorptionsmaximum bei 268 nm, was dem von Etherolensäure entspricht (siehe Abb. 3).

### Beispiel 7: Nachweis der Divinylether durch GC/MS

Die Produkte, die durch die Umsetzung von 13-HPOD bzw. -HPOT und 9-HPOD mit der rekombinanten 9/13-DES erhalten wurden, wurden durch Normalphasen-HPLC zunächst von anderen Oxylipinen abgetrennt, bevor die Trennung der Divinylether durch die Umkehrphasen-HPLC erfolgte.

Für alle HPLC-Verfahren wurde ein Hewlett Packard HPLC-System, welches einen Dioden-Array-Detektor enthielt (Agilent 1100, Waldbronn), bei einer Säulentemperatur von 25°C durchgeführt.

Die Normalphasen-HPLC der Divinylether wurde mit folgenden Parametern durchgeführt:
- Säule:: 150 x 2,1mm, 5 µm Partikelgröße, Zorbax RX-SIL von Hewlett Packard
- Laufmittel:: Hexan/Isopropanol/Eisessig (100:1:0,1, v/v/v)
- Injektionsvolumen:: 25 µl
- Fluss:: 0,2 ml/min
- Laufschema:: isokratisch von 0 bis max. 30 min je nach Alter des Laufmittels
- Standard:: dinor CnA, CnA, CA
- Laufmittel:: Hexan/Isopropanol/TFA (100:1:0,1, v/v/v)
- Fluss:: 0,1 ml/min
- Laufschema:: isokratisch von 0 bis 50 min

Anhand der Standards wurden die Retentionszeiten der Divinylether ermittelt und anschließend das Eluat zu der entsprechenden Zeit aufgefangen.

Nachdem die Divinylether durch die Normal-Phasen-HPLC von anderen Oxylipinen getrennt worden sind, erfolgte die Trennung der Divinylether durch die Umkehrphasen-HPLC mit folgenden Parametern:
- Säule:: 250 x 2 mm, 5 µm Partikelgröße, EC 250/2 NUCLEOSIL 120-5 C-18 von Macherey-Nagel (Düren)
- Laufmittel:: Methanol/H₂O/Eisessig (90 : 10 : 0,1, v/v/v)
- Injektionsvolumen:: 80 µl
- Fluss:: 0,18 ml/min
- Laufschema:: isokratisch von 0 bis 25 min
- Standard:: dinor CnA, Cna, Ca

Eine GC/MS-Analyse der so erhaltenen Divinylether ist erst nach Derivatisierung zu den entsprechenden Methylestern möglich. Dazu wurden 20 µl der Probe in 380 µl Methanol aufgenommen und mit 10 µl EDAC-Lösung (Sigma, Deisenhofen) versetzt. Anschließend wurde die Probe für 2 Stunden bei Raumtemperatur geschüttelt. Nach Zugabe von 200 µl 0,1 M Tris/HCl, pH 7,5 wurden die methylierten Fettsäuren zweimal mit 1 ml Hexan extrahiert, bevor 1 ml Hexan hinzugegeben und gründlich geschüttelt wurde. Anschließend wurden die Phasen durch 5minütige Zentrifugation bei Raumtemperatur mit 13100 g getrennt. Die Hexanphasen wurden vereinigt und unter Stickstoffstrom eingedampft. Für die Analyse in der Gaschromatographie wurden die Fettsäuren in 10 µl Acetonitril gelöst.

Nach der Methylierung erfolgte die GC-Analyse der Fettsäuren und deren Derivate mit einer GC von Agilent Technologies 6890 Series. Dazu wurden folgende Parameter verwendet:
- Säule: DB 23 von Scientific, Länge: 30 m, Durchmesser: 0,25 mm
Filmdicke: 0,25 µm
- Fluss: 1 ml/min
- Injektortemperatur: 150°C
- Injektionsmodus: Splitmessung
150°C für eine Minute → 4°C/min auf 200°C→ 5°C/min auf
- Temperaturprogramm: 250°C und für 5 Minuten konstant gehalten

Für die Massenspektroskopie wurde ein MS 5973 von Agilent Technologies mit den folgenden Parametern verwendet:

| | |
|---|---|
| Transferleitungstemperatur | 260°C |
| Ionisierungsart | Elektronenstoßionisierung |
| Ionisierungstärke | 70 eV |
| Quellentemperatur | 230°C |
| Scanmodus | Vollscanmodus |

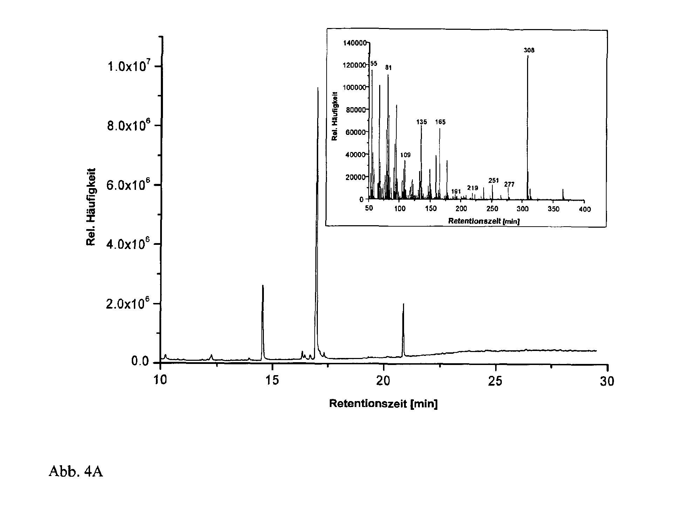

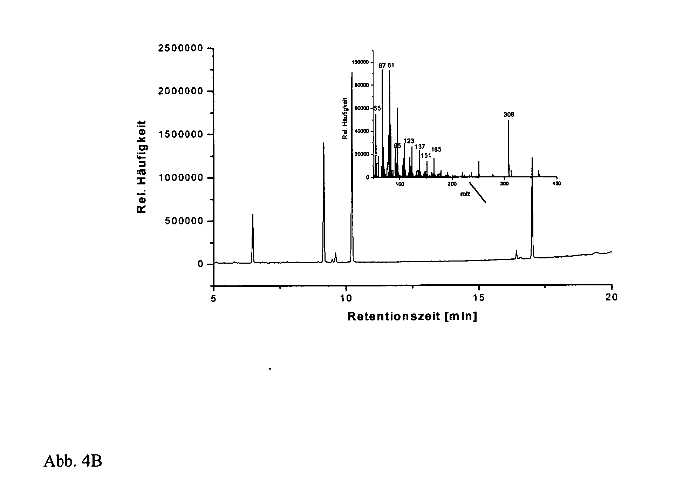

Im Falle des aus 13-HPOD gebildeten Produkts Etherolsäure (EA) zeigte sich das in Abb. 4A dargestellte Elutionsprofil. Durch das Massenspektrum konnte die detektierte Substanz als EA identifiziert werden. Im Falle des aus 9-HPOD gebildeten Produkts Colnelsäure (CA) zeigte sich das in Abb. 4B dargestellte Elutionsprofil. Durch das Massenspektrum konnte die detektierte Substanz als CA identifiziert werden.

### Beispiel 8: Herstellung und Nachweis von C₆-Aldehyden aus Etherolsäure

Für die Herstellung von C₆-Aldehyden aus Etherolsäure wurden 10 µg Etherolsäure in 250 µl Methanol mit 750 µl 20% HCl versetzt.

Die so entstandenen Aldehyde wurden durch die Behandlung mit dem Derivatisierungsreagens DNPH (1 mg in 1 ml 1 N HCl) in die entsprechenden Hydrazone umgewandelt, die durch HPLC identifiziert werden können. Die dadurch entstandenen Dinitrophenylhydrazone wurden nach Schütteln für 1 Stunde dreimal mit 1 ml Hexan extrahiert. Die vereinigten Hexanphasen wurden im Stickstoffstrom eingedampft und in 100 µl Laufmittel A aufgenommen.

Die derivatisierten Aldehyde wurden in einer Umkehrphasen-HPLC mit den folgenden Parametern nachgewiesen:
Säule: 250 x 2 mm, 5 µm Partikelgröße, EC 250/2 NUCLEOSIL 120-5 C-18 von Macherey-Nagel (Düren)
Laufmittel: A: Acetonitril/Wasser/Eisessig (50:50:0,1, v/v/v)
B: Acetonitril/Eisessig (100:0,1, v/v/v)
Injektionsvolumen: 50 µl
Standard: 2E-Pentenal, Hexenal, Hexanal, Nonadienal, Nonenal
Die Proben wurden nach dem Eindampfen unter Stickstoffstrom in 50 µl Acetonitril/Wasser (60/40, v/v) resuspendiert
Laufschema:

| Zeit [min] | % Laufmittel B | Fluss [ml/min] |
|---|---|---|
| 0 | 20 | 0,3 |
| 15 | 20 | 0,3 |
| 20 | 40 | 0,3 |
| 33,4 | 60 | 0,3 |
| 50 | 60 | 0,3 |
| 55-60 | 20 | 0,3 |

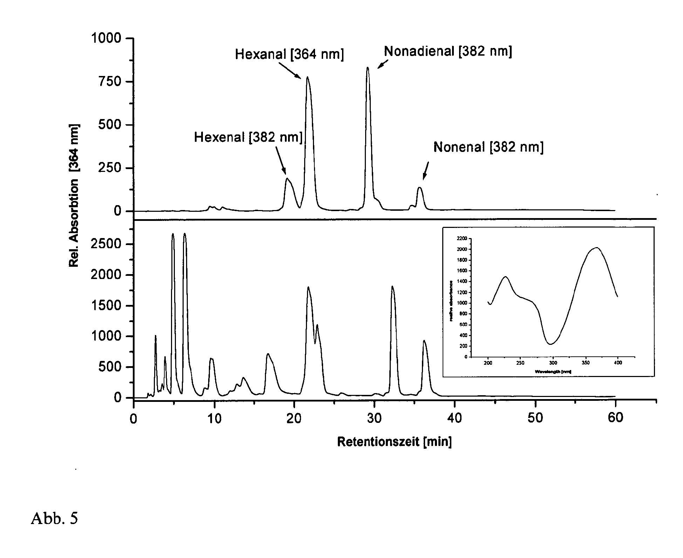

Mit Hilfe der Standards konnte das Aldehyd, das durch die Spaltung von Etherolsäure mit Säure entsteht, als Hexanal, das bei 364 nm absorbiert, identifiziert werden (siehe Abb. 5).

### Beispiel 9: Bestimmung des pH-Optimums der 9/13-DES

Das pH-Optimum wurde mittels einer Endpunktbestimmung mit Hilfe der Normal-Phasen-HPLC und der Umkehr-Phasen-HPLC ermittelt. Es wurden sowohl 9-HPOD als auch 13-HPOD als Substrate der DES verwendet. Für die Bestimmung des pH-Optimums wurde die Umsetzung bei pH-Werten von 4,5 bis 8,5 in Schritten von 0,5 pH-Einheiten durchgeführt. Für pH 7 wurden zweimal drei Messungen durchgeführt, da für diesen pH-Wert sowohl ein 50 mM Natrium-Phosphatpuffer als auch ein 50 mM Tris/HCl-Puffer verwendet wurde. Für die Umsetzungen wurden zwischen 25 und 80 nmol der Hydroperoxide eingesetzt. Von dem einzelnen Hydroperoxid wurde dabei immer die gleiche Menge für jede Umsetzung eingesetzt. Parallel zu den Proben für den pH-Optimumstest wurden drei Ansätze mit Zelllysat des leeren Vektors in *E. coli* mit 13-HPOD inkubiert. Die Leervektorkontrolle zeigte keine Divinylether-Bildung, es konnte nur das Hydroperoxid nachgewiesen werden.

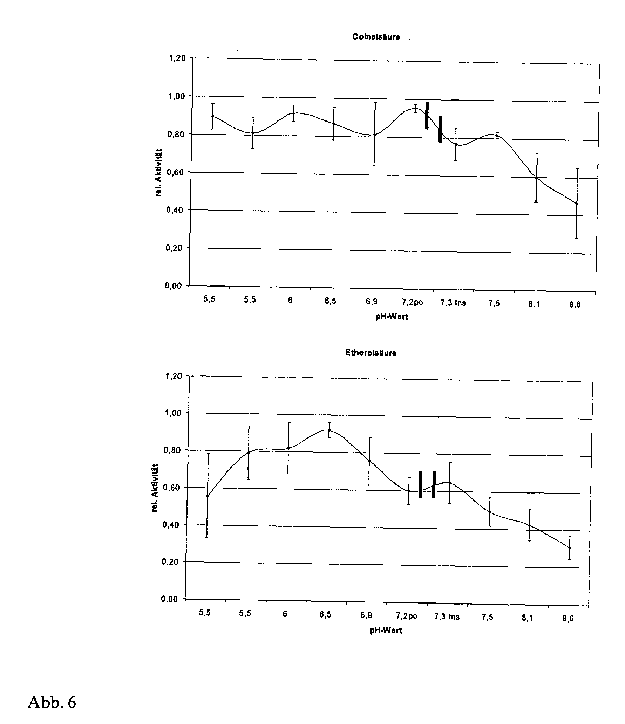

Für 13-HPOD als Substrat wurde ein sehr breites pH-Spektrum ermittelt mit einem Optimum zwischen pH 5,5 und 6,9 (siehe Abb. 6), was in etwa dem pH-Optimum der 9-DES aus S. *tuberosum* entspricht (Stumpe et al., 2001, *vide supra).* Für 9-HPOD war keine Bestimmung eines pH-Optimums möglich, da das Substrat scheinbar über den gesamten pH-Bereich von pH 5,5 bis 8,6 gleich gut umgesetzt wurde (siehe Abb. 6).

### Beispiel 10: Photometrische Bestimmung der Substratspezifität der 9/13-DES

Für die Bestimmung der Substratspezifität wurde die 9/13-Divinylethersynthase aus Knoblauch aus einer Expressionskultur aufgereinigt. Dazu wurde zunächst eine 400 ml Expressionskultur unter den schon vorher beschriebenen Bedingungen angeimpft und angezogen. Die Zellen wurden nach zweitägiger Inkubation durch 15-minütige Zentrifugation mit 4000 g bei 4°C sedimentiert. Das Sediment wurde in 20 ml 50 mM Na-Phosphat, pH 8, resuspendiert und mit Ultraschall (Sonopuls GM 70, Bandelin, Berlin) für 5-Mal eine Minute bei 50 % Leistung und 50 % Impuls aufgeschlossen. Die Zelltrümmer wurden 15 min bei 4000 x g sedimentiert und aus dem Überstand wurden durch eine weitere Zentrifugation (1 h bei 100000 x g) die Zellmembranen sedimentiert. Das Membransediment wurde für 1 h in 50 mM Na-Phosphat, pH 8, mit 1M NaCl und 0,1 % (v/v) TritonX-100 inkubiert, um die Membranproteine zu solubilisieren. Anschließend wurde erneut 1 h bei 100000 x g zentrifugiert. Der Überstand wurde mit Talon Metal Affinity Resin (Clontech, Palo Alto, USA) (200 µl Bettvolumen) über Nacht bei 4 °C geschüttelt. Danach wurde das Material auf eine Leersäule (T.J. Baker, Phillipsburg, N.J., USA) gegeben. Die Elution erfolgte mit 3 ml 50 mM Tris/HCl pH 6,3, 10 % Glycerin, 0,1 % Tween 20, 0,5 M NaCl und 0,1 M EDTA. Durch eine PD-10 Säule (Amersham pharmacia biotech, UK) wurde das Protein in 50 mM Na-Phosphat-Puffer pH 8 umgepuffert.

Das so aufgereinigte Protein wurde für die Tests im Photometer eingesetzt. Die Messungen erfolgten bei Raumtemperatur und es wurde immer bei der Wellenlänge des eingesetzten Hydroperoxids (234 bzw. 236 nm) bei einer Konzentration 100 µM für eine Minute die Abnahme der Extinktion aufgezeichnet. Die Messungen erfolgten in einer 100 µl Quarzküvette. Die Reaktion wurde durch die Zugaben von 1 µl der Proteinlösung gestartet.

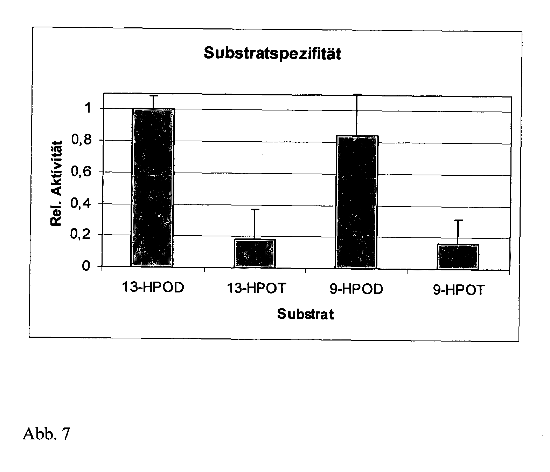

Es zeigte sich, dass die erfindungsgemäße 9/13-DES bevorzugt 13-HPOD und in etwas geringerem Maße 9-HPOD umsetzt, während 9-HPOT und 13-HPOT wesentlich weniger stark umgesetzt werden (siehe Abb. 7).

### Beispiel 11: Agrobacterium tumefaciens-vermittelte Transformation von Pflanzen

Die stabile Transformation von Pflanzen mit Hilfe von *A. tumefaciens* erfolgt beispielsweise nach folgendem Protokoll:

Die kodierende Sequenz der 9/13-DES wird aus dem Expressionsvektor pQE 30 mit geeigneten Restriktionsenzymen herausgeschnitten und im Agarosegel gereinigt. Das Fragment wird in den vorgeschnittenen pUC 1 8-Entry-Vektor ligiert und zur Vermehrung in *E. coli* XLI-Blue transformiert.

Der so erhaltene pUC18-Entry-AsDES-Vektor wird mit dem pCAMBIA-Vektor und dem Clonase-Enzym Mix von Invitrogen für 60 min bei 25°C inkubiert. Danach erfolgt die Zugabe von 2µl Proteinase-K-Lösung und eine Inkubation bei 37°C von 10 min. 5µl dieses Ansatzes werden in *E. coli* XLI-Blue transformiert. Der so erhaltene pCAMBIA-AsDES-Vektor besitzt den CaMV 35S-Promotor und eine BASTA-Resistenz zur Selektionierung der Pflanzen.

Zur Transformation in Arabidopsis wird von positiv transformierten *A. tumefaciens* eine 200 ml Übernachtkultur angesetzt und bei 28°C geschüttelt. Die Bakterienlösung wird bei 4°C für 20 min abzentrifugiert. Das Pellet wird in 200ml 5% Saccharoselösung aufgenommen und 100µl Silvette dazugegeben. Die Arabidopsispflanzen werden kurz in diese Lösung getaucht und über Nacht mit einer Haube abgedeckt. Die Samen der so transformierten Pflanzen wird vereinigt und auf Erde ausgesät.

### Abbildungen:

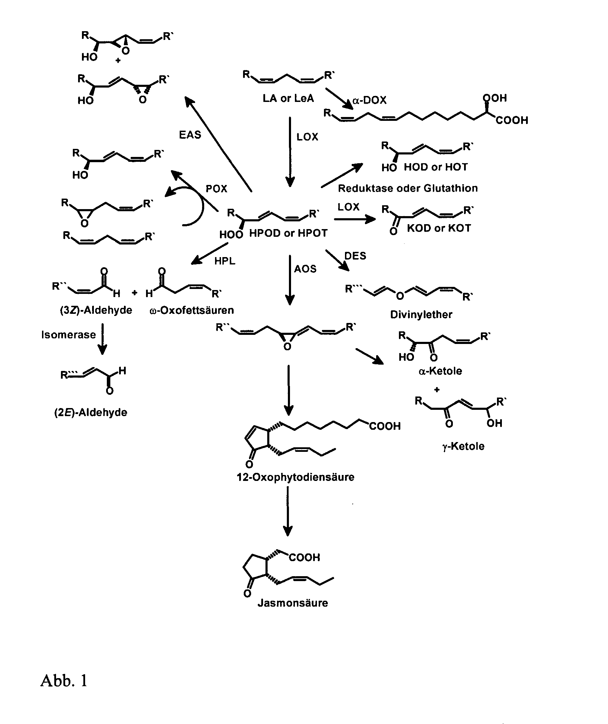

Abb. 1: Reaktionswege der Oxylipin-Biosynthese. Linolensäure und Linolsäure werden durch Lipoxygenase (LOX) in Hydroperoxide umgesetzt. Die Hydroperoxidprodukte werden anschließend durch Allenoxidsynthase (AOS), Hydroperoxidlyase (HPL) und Divinylethersynthase (DES) zu verschiedenen Oxylipin-Intermediaten oder Endprodukten metabolisiert.
Abb. 2: Analyse der 9/13-DES-Expression durch SDS-PAGE-Analyse und anschließende Coomassie Brilliant Blue-Färbung sowie Western-Blot-Analyse (rechts). Die Spuren 1 - 3 zeigen die Proteine der mit Leervektor transformierten Zellen vor der Induktion mit IPTG (1), einen Tag nach der Induktion (2) und zwei Tage nach der Induktion (3). Daneben zeigen die Spuren 4 - 6 die Proteine der mit dem Expressionsvektor transformierten Zellen vor der Induktion mit IPTG (4), einen Tag nach der Induktion (5) und zwei Tage nach der Induktion (6). Der Pfeil zeigt die Banden des rekombinanten Proteins bei ca. 55 kDa.
Abb. 3: Analyse der Reaktionsprodukte aus dem Aktivitätstest der 9/13-DES mittels Reversphasen-HPLC. Die Produkte wurden anhand ihrer Absorption bei 268 und 252 nm detektiert. Das Signal bei 29,0 Minuten hat das in Fenster B dargestellte Absorptionsmaximum von Etherolensäure, nämlich 268 nm. In der Probe konnte kein restliches Substrat detektiert werden.
Abb. 4:
   A) GC/MS-Chromatogramm des Produktes aus 13-HPOD; das Signal bei 17 Minuten hat das im Fenster dargestellte Massenspektrum, das es als Signal für die Etherolsäure identifiziert. Die Peaks, die zur Charakterisierung von EA benutzt wurden, sind mit ihren Molekulargewichten beschriftet.
   B) GC/MS-Chromatogramm des Produktes aus 9-HPOD; das Signal bei 17 Minuten hat das im Fenster dargestellte Massenspektrum, das es als Signal für die Colnelsäure identifiziert. Die Peaks, die zur Charakterisierung von CA benutzt wurden, sind mit ihren Molekulargewichten beschriftet
   .
Abb. 5: HPLC-Spektrogramm des mit DNPH derivatisierten Aldehyds, der bei der Spaltung von Etherolsäure mit Säure entsteht (Fenster B). Anhand von verschiedenen Standards (Fenster A) konnte er als Hexanal, welches bei 364 nm absorbiert, nachgewiesen werden (Fenster C).
Abb. 6: pH-Optimum der 9/13-DES für zwei Substrate (9-HPOD und 13-HPOD). Verglichen wurde die Produktbildung von EA aus 13-HPOD und von CA aus 9-HPOD. Für die niedrigen pH-Werte von 5,5 bis 7,2 wurde 50 mM Na-Phosphat-Puffer eingesetzt; von 7,3 bis 8,6 wurden 50 mM Tris/HCl verwendet. Die Trennlinien markieren den Überlappungsbereich. In den Abbildungen ist der Standardfehler angegeben.
Abb. 7: Relative Substratspezifität der 9/13-DES für die Substrate 9- und 13-HPOD sowie 9- und 13-HPOT.

Die Substratspezifität der erfindungsgemäßen 9/13-DES wurde photometrisch bestimmt. Der Standardfehler ist angegeben.

## Patentansprüche

1. Isoliertes Nukleinsäuremolekül, enthaltend eine Nukleinsäuresequenz ausgewählt aus der Gruppe bestehend aus:
i) DNA-Sequenzen umfassend Nukleotidsequenzen, die von SEQ ID No. 1 oder Fragmenten dieser Sequenz kodiert werden,
ii) DNA-Sequenzen umfassend Nukleotidsequenzen, die für ein Protein mit der in SEQ ID No. 2 angegebenen Aminosäuresequenz oder Fragmente davon kodieren,
iii) DNA-Sequenzen umfassend Nukleotidsequenzen, die zu der in SEQ ID No. 1 angegebenen Nukleotidsequenz eine Sequenzidentität von mindestens 60% aufweisen, und/oder
iv) DNA-Sequenzen umfassend Nukleotidsequenzen, die unter stringenten Bedingungen mit einem komplementären Strang einer Nukleotidsequenz von i) bis iii) hybridisieren,
die für ein Protein mit der Aktivität einer 9/13-Divinylethersynthase kodiert.

2. Nukleinsäuremolekül nach Anspruch 1, wobei die Nukleinsäuresequenz aus *Allium sativum* stammt.

3. Rekombinantes Protein mit der Aktivität einer 9/13-Divinylethersynthase, kodiert durch eine Nukleinsäuresequenz wie in Anspruch 1 oder 2 angegeben, oder ein funktionell aktives Fragment davon.

4. Rekombinantes Nukleinsäuremolekül, umfassend eine Nukleinsäuresequenz wie in Anspruch 1 oder 2 angegeben sowie geeignete Regulations- und/oder Signalsequenzen.

5. Rekombinantes Nukleinsäuremolekül nach Anspruch 4, wobei die Regulations- und Signalsequenzen die Expression in prokaryontischen Zellen steuern.

6. Rekombinantes Nukleinsäuremolekül nach Anspruch 4, wobei die Regulations- und Signalsequenzen die Expression in Pflanzenzellen steuern.

7. Mikroorganismus enthaltend ein rekombinantes Nukleinsäuremolekül nach Anspruch 4 oder 5.

8. Mikroorganismus nach Anspruch 7, der einen gegenüber Wildtyp-Organismen erhöhten Gehalt eines Proteins nach Anspruch 3 aufweist.

9. Transgene Pflanzenzelle, enthaltend ein rekombinantes Nukleinsäuremolekül nach Anspruch 4 oder 6.

10. Transgene Pflanzenzelle nach Anspruch 9, die einen gegenüber Wildtypzellen erhöhten Gehalt eines Proteins nach Anspruch 3 aufweist.

11. Verfahren zur Herstellung von C₆- und/oder C₉-Aldehyden bzw. C₆- und/oder C₉-Alkoholen bzw. Estern der C₆- und/oder C₉-Alkohole aus mehrfach ungesättigten Fettsäuren der Kettenlänge C₁₈, die zumindest Doppelbindungen an den Positionen Δ9 und Δ12 aufweisen, umfassend die folgenden Schritte:
a) die Bereitstellung eines rekombinanten Nukleinsäuremoleküls nach einem der Ansprüche 4 bis 6;
b) die Übertragung des Nukleinsäuremoleküls aus a) auf eukaryontische oder prokaryontische Zellen,
c) die Expression der rekombinanten 9/13-Divinylethersynthase in den Zellen;
d) die Umsetzung von 9- und/oder 13-Hydroperoxiden der Fettsäuren mit 9/13-Divinylethersynthase zu Divinylethern;
e) die Umsetzung der Divinylether zu (2E)-C₆- und/oder (2E)-C₉-Aldehyden;
f) gegebenenfalls die Reduktion der C₆- und/oder C₉-Aldehyde zu C₆- und/oder C₉₋Alkoholen;
g) gegebenenfalls die Umsetzung der C₆- und/oder C₉-Alkohole aus Schritt e) mit einer Säure, insbesondere Essigsäure, zu einem entsprechenden Ester.

12. Verfahren nach Anspruch 11, wobei zusätzlich ein rekombinantes Nukleinsäuremolekül bereit gestellt wird, das für ein pflanzliches oder mikrobielles Protein mit der biologischen Aktivität einer 9- oder 13-Lipoxygenase kodiert.

13. Verfahren nach Anspruch 12,
wobei das Protein mit der biologischen Aktivität einer 9- oder 13-Lipoxygenase aus *Solanum tuberosum* bzw. Sojabohne stammt.

14. Verfahren nach einem der Ansprüche 11 bis 13,
wobei die C₁₈-Fettsäuren ausgewählt werden aus der Gruppe bestehend aus α-Linolensäure und Linolsäure.

15. Verfahren nach einem der Ansprüche 11 bis 14, wobei es sich bei den eukaryotischen Zellen um Pflanzenzellen handelt.

16. Verfahren nach Anspruch 15,
wobei im Anschluss an die Übertragung der Nukleinsäuresequenz auf pflanzliche Zellen aus den transgenen Zellen vollständige Pflanzen regeneriert werden, die als Produktionsstätte für Divinylether sowie C₆- und/oder C₉-Aldehyde bzw. C₆- und/oder C₉-Alkohole bzw. Ester der C₆- und/oder C₉-Alkohole dienen.

17. Verfahren nach Anspruch 15,
wobei transgene Pflanzenzellen kultiviert und als Produktionsstätte für Divinylether sowie C₆- und/oder C₉-Aldehyde bzw. C₆- und/oder C₉-Alkohole bzw. Ester der C₆- und/oder C₉₋Alkohole genutzt werden.

18. Verfahren nach einem der Ansprüche 11 bis 14,
wobei die transformierten Zellen Bakterienzellen, Hefezellen und Algenzellen umfassen und die Kultivierung der Zellen und die Herstellung der C₉-Aldehyde bzw. C₉-Alkohole bzw. Ester der C₉-Alkohole in einem Reaktor erfolgt.

19. Verfahren nach einem der Ansprüche 11 bis 18,
wobei die Umsetzung der Divinylether zu Aldehyden in Schritt e) von Anspruch 11 durch mildes Ansäuern erfolgt.

20. Verfahren nach einem der Ansprüche 11 bis 19,
wobei die Reduktion in Schritt f) von Anspruch 11 enzymatisch, vorzugsweise mit Alkoholdehydrogenase erfolgt.

21. Verfahren nach einem der Ansprüche 11 bis 19,
wobei die Reduktion in Schritt f) von Anspruch 11 chemisch, vorzugsweise mit Natriumborhydrid erfolgt.

22. Verfahren zur Herstellung von C₆- und/oder C₉-Aldehyden bzw. C₆- und/oder C₉-Alkoholen bzw. Estern der C₆- und/oder C₉-Alkohole, umfassend folgende Komponenten:
a) mindestens eine mehrfach ungesättigte C₁₈-Fettsäure, die zumindest Doppelbindungen an den Positionen Δ9 und Δ12 aufweist;
b) 9/13-Divinylethersynthase;
c) gegebenenfalls ein Reduktionsmittel; sowie,
d) gegebenenfalls eine Säure, insbesondere Essigsäure.

23. Verfahren nach Anspruch 22,
wobei die C₁₈-Fettsäuren ausgewählt werden aus der Gruppe, bestehend aus α-Linolensäure und Linolsäure.

24. Verfahren nach Anspruch 22 oder 23,
wobei eine rekombinante 9/13-Divinylethersynthase aus *Allium sativum* verwendet wird.

25. Verfahren nach einem der Ansprüche 22 bis 24,
wobei ein enzymatisches Reduktionsmittel, vorzugsweise eine Alkoholdehydrogenase verwendet wird.

26. Verfahren nach einem der Ansprüche 22 bis 25,
wobei ein chemisches Reduktionsmittel, vorzugsweise Natriumborhydrid verwendet wird.

27. Verfahren nach einem der Ansprüche 22 bis 26, zusätzlich umfassend 9-Lipoxygenase.

28. Verfahren nach Anspruch 27,
wobei eine rekombinante 9-Lipoxygenase aus *Solanum tuberosum* verwendet wird.

29. Verwendung von 9/13-Divinylethersynthase nach Anspruch 3 zur Herstellung von C₆- und/oder C₉-Aldehyden bzw. C₆- und/oder C₉-Alkoholen bzw. Estern der C₆- und/oder C₉-Alkohole.

30. Verfahren zur Herstellung von transgenen Pflanzenzellen bzw. transgenen Pflanzen mit erhöhter Pathogenresistenz, umfassend die folgenden Schritte:
a) Herstellen eines rekombinanten Nukleinsäuremoleküls, das folgende Sequenzen umfasst:
- regulatorische Sequenzen eines in Pflanzenzellen aktiven Promotors;
- operativ daran gebunden eine Nukleinsäuresequenz wie in Anspruch 1 oder 2 angegeben; und
- optional operativ daran gebunden regulatorische Sequenzen, die als Transkriptions-, Terminations- und/oder Polyadenylierungssignale in Pflanzenzellen dienen können;
b) Übertragen des Nukleinsäuremoleküls aus a) auf pflanzliche Zellen; und
c) gegebenenfalls die Regeneration von Pflanzen aus den transformierten Pflanzenzellen.

31. Transgene Pflanzenzellen, hergestellt nach einem Verfahren nach Anspruch 30.

32. Transgene Pflanzen, enthaltend eine Pflanzenzelle nach Anspruch 31 oder durch ein Verfahren nach Anspruch 30, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

33. Verwendung eines Nukleinsäuremoleküls nach Anspruch 1 oder 2 zur Herstellung von transgenen Pflanzen und Pflanzenzellen mit erhöhter Pathogenresistenz.

34. Verwendung von durch die Aktivität der 9/13-Divinylethersynthase nach Anspruch 3 *in vitro* hergestellten Divinylethern zur Applikation auf Pflanzen, um eine erhöhte Pathogenresistenz zu erzeugen.

35. Zusammensetzung, die Pathogenresistenz vermittelt, umfassend mindestens eine 9- oder 13-Divinyletherfettsäure und einen Trägerstoff.


SEQUENCE LISTING

<110>  Georg-August-Universität Göttingen

<120>  Verfahren zur Herstellung von C6- und/oder C9-Aldehyden und
       -Alkoholen in Pflanzen durch 9/13-Divinylethersynthase

<130>  U 7076

<140>  DE-
<141>  2004-07-28

<160>  8

<170>  PatentIn version 3.3

<210>  1
<211>  1419
<212>  DNA
<213>  Allium sativum

<400>  1
atgtccacct ccaacggcag cacggaaaac atccagaaac ccctcagaaa aatccctgat      60

ataaccggca ccccaatcct aactgccatc aaagaccgcc tcgacttctt ctacaaccaa     120

ggccaatacg agtacttcca atccagagta aagaaaaaca actcaaccat cctgcgcatg     180

aacatgatcc cgggcccctt cgcctccaac ccaaaaatag tcgccctctg cgacgctgca     240

tctttcccga ccctcttcga cccttccaag gtctccaaag ttaactccct cacagggaac     300

tacatgcccg ctctatcatt caccggcggg taccgggttt gtgcctacct cgatccgtcc     360

gagcccaccc acactaaaat caagcaggtg ttcttcaatg cgcaagccgc taaaaaagac     420

actttcatcc ccactttcgt gagcactttc aattccatgt tcgataagat ggatgccgag     480

gtggaaagca agaagaaggc ggagtttacc aagttcaacg aggcggcagt gtttgagttt     540

gtcgggctgg ctctggttgg gccgaagccc gcccgtgagg tttttgatag tgcgaagaag     600

tccgtgttct ttcagtttca tcctttcatt acggcggggc ttccggctct cgtcgaagaa     660

ctcgctttcc atatgtttcc gttcccgagc tttgttgcga agtccagtta taagatattg     720

tatgagtact tcagtactgg tgggtcttgg atacttgaca atgctgaaga gatcggttta     780

tcaagagaag aagcaatcca ccacctaata ttcacatggg caataaacgc ctacctcggc     840

atacgaacct gtctaatgcg cctattcaaa tggatcgtgg catcaggacc ggatctccaa     900

gaaaaactag cgcgagaagt acgttcagta gtccgttcgg aggaaggcaa gatcacattc     960

gcgggcattg agaaaatgga acttgtaaaa tcggtcgcct atgagtcctt ccggttcgac    1020

ccgccggtcc aggtccagta tggcaccgcg aaatccgacc tcatcataga gagccacgac    1080

ggcaagtacc aagtgaagaa aggggaaatg ttgtgtggtt tccaaccaat ggcgacccgt    1140

gacccgaagg tatttgaccg agcagacgag tttgtgcccg ataggttcat gggagatggt    1200

aagaagttgg tgaagcacgt gttgtgggct aacgggtacg gaacggatgc tccgaaagcg    1260

gatgataaga tatgtgctgg gaaggactta ggggttttgg ttgggaggtt gctgattgct    1320

gttatgttct tgaggtatga taagattggt ggggtggttg ggaagaccat ggaggaggtt    1380

gatgtcattg tgaatgagct gaccaaggta gcagtctga                                1419

<210>  2
<211>  472
<212>  PRT
<213>  Allium sativum

<400>  2

Met Ser Thr Ser Asn Gly Ser Thr Glu Asn Ile Gln Lys Pro Leu Arg
1               5                   10                  15

Lys Ile Pro Asp Ile Thr Gly Thr Pro Ile Leu Thr Ala Ile Lys Asp
            20                  25                  30

Arg Leu Asp Phe Phe Tyr Asn Gln Gly Gln Tyr Glu Tyr Phe Gln Ser
        35                  40                  45

Arg Val Lys Lys Asn Asn Ser Thr Ile Leu Arg Met Asn Met Ile Pro
    50                  55                  60

Gly Pro Phe Ala Ser Asn Pro Lys Ile Val Ala Leu Cys Asp Ala Ala
65                  70                  75                  80

Ser Phe Pro Thr Leu Phe Asp Pro Ser Lys Val Ser Lys Val Asn Ser
                85                  90                  95

Leu Thr Gly Asn Tyr Met Pro Ala Leu Ser Phe Thr Gly Gly Tyr Arg
            100                 105                 110

Val Cys Ala Tyr Leu Asp Pro Ser Glu Pro Thr His Thr Lys Ile Lys
        115                 120                 125

Gln Val Phe Phe Asn Ala Gln Ala Ala Lys Lys Asp Thr Phe Ile Pro
    130                 135                 140

Thr Phe Val Ser Thr Phe Asn Ser Met Phe Asp Lys Met Asp Ala Glu
145                 150                 155                 160

Val Glu Ser Lys Lys Lys Ala Glu Phe Thr Lys Phe Asn Glu Ala Ala
                165                 170                 175

Val Phe Glu Phe Val Gly Leu Ala Leu Val Gly Pro Lys Pro Ala Arg
            180                 185                 190

Glu Val Phe Asp Ser Ala Lys Lys Ser Val Phe Phe Gln Phe His Pro
        195                 200                 205

Phe Ile Thr Ala Gly Leu Pro Ala Leu Val Glu Glu Leu Ala Phe His
    210                 215                 220

Met Phe Pro Phe Pro Ser Phe Val Ala Lys Ser Ser Tyr Lys Ile Leu
225                 230                 235                 240

Tyr Glu Tyr Phe Ser Thr Gly Gly Ser Trp Ile Leu Asp Asn Ala Glu
                245                 250                 255

Glu Ile Gly Leu Ser Arg Glu Glu Ala Ile His His Leu Ile Phe Thr
            260                 265                 270

Trp Ala Ile Asn Ala Tyr Leu Gly Ile Arg Thr Cys Leu Met Arg Leu
        275                 280                 285

Phe Lys Trp Ile Val Ala Ser Gly Pro Asp Leu Gln Glu Lys Leu Ala
    290                 295                 300

Arg Glu Val Arg Ser Val Val Arg Ser Glu Glu Gly Lys Ile Thr Phe
305                 310                 315                 320

Ala Gly Ile Glu Lys Met Glu Leu Val Lys Ser Val Ala Tyr Glu Ser
                325                 330                 335

Phe Arg Phe Asp Pro Pro Val Gln Val Gln Tyr Gly Thr Ala Lys Ser
            340                 345                 350

Asp Leu Ile Ile Glu Ser His Asp Gly Lys Tyr Gln Val Lys Lys Gly
        355                 360                 365

Glu Met Leu Cys Gly Phe Gln Pro Met Ala Thr Arg Asp Pro Lys Val
    370                 375                 380

Phe Asp Arg Ala Asp Glu Phe Val Pro Asp Arg Phe Met Gly Asp Gly
385                 390                 395                 400

Lys Lys Leu Val Lys His Val Leu Trp Ala Asn Gly Tyr Gly Thr Asp
                405                 410                 415

Ala Pro Lys Ala Asp Asp Lys Ile Cys Ala Gly Lys Asp Leu Gly Val
            420                 425                 430

Leu Val Gly Arg Leu Leu Ile Ala Val Met Phe Leu Arg Tyr Asp Lys
        435                 440                 445

Ile Gly Gly Val Val Gly Lys Thr Met Glu Glu Val Asp Val Ile Val
    450                 455                 460

Asn Glu Leu Thr Lys Val Ala Val
465                 470

<210>  3
<211>  22
<212>  DNA
<213>  Artificial

<220>

<223>  Primer CF44_sonder

<400>  3
gcttcaccag cttcttgccg tc                                                22


<210>  4
<211>  24
<212>  DNA
<213>  Artificial

<220>
<223>  Primer CF44_sondeu

<400>  4
ggcgtttacg aagtttaacg aggc                                              24


<210>  5
<211>  24
<212>  DNA
<213>  Artificial

<220>
<223>  Primer CF44_5race

<400>  5
gcctcgttaa acttcgtaaa cgcc                                              24


<210>  6
<211>  22
<212>  DNA
<213>  Artificial

<220>
<223>  Primer CF44_3race

<400>  6
gacggcaaga agctggtgaa gc                                                22


<210>  7
<211>  29
<212>  DNA
<213>  Artificial

<220>
<223>  Primer AsDesBamHIuni

<400>  7
ggatccatgt ccacctccaa cggcagcac                                         29


<210>  8
<211>  30
<212>  DNA
<213>  Artificial

<220>
<223>  Primer AsDesPstIrev2

<400>  8
ctgcagtcag actgctacct tggtcagctc                                        30